(19) Europäisches Patentamt
European Patent Office
Office européen des brevets

(11) EP 1 782 804 B1

(12) **EUROPEAN PATENT SPECIFICATION**

(45) Date of publication and mention of the grant of the patent:
**28.12.2011 Bulletin 2011/52**

(21) Application number: **05767088.7**

(22) Date of filing: **28.07.2005**

(51) Int Cl.:
*A61K 31/133* (2006.01) *A61K 31/192* (2006.01)
*A61K 31/22* (2006.01) *A61K 31/40* (2006.01)
*A61K 45/06* (2006.01) *A61P 37/06* (2006.01)
*C07D 207/335* (2006.01) *A61K 31/137* (2006.01)
*A61K 31/166* (2006.01) *A61K 31/366* (2006.01)

(86) International application number:
**PCT/JP2005/013843**

(87) International publication number:
**WO 2006/011554 (02.02.2006 Gazette 2006/05)**

(54) **MEDICINAL COMPOSITION, COMPRISING A HMG-CoA REDUCTASE INHIBITOR AND AN AMINO ALCOHOL DERIVATIVE, AS IMMUNOSUPPRESSANT**

MEDIZINISCHE ZUSAMMENSETZUNG, ENTHALTEND EIN HMG-CoA REDUKTASE INHIBITOR UND EIN AMINOALKOHOL DERIVAT, ALS IMMUNSUPPRESSIVUM

COMPOSITION MÉDICINALE, COMPRENANT UN INHIBITEUR DE LA HMG-CoA REDUCTASE ET UN DÉRIVÉ D'AMINOALCOOL, UTILE EN TANT QU'IMMUNOSUPPRESSEUR

(84) Designated Contracting States:
**AT BE BG CH CY CZ DE DK EE ES FI FR GB GR HU IE IS IT LI LT LU LV MC NL PL PT RO SE SI SK TR**

(30) Priority: **29.07.2004 JP 2004222420**

(43) Date of publication of application:
**09.05.2007 Bulletin 2007/19**

(73) Proprietor: **Daiichi Sankyo Company, Limited**
**Tokyo 103-8426 (JP)**

(72) Inventors:
- **NISHI, Takahide**
  **c/o Sankyo Compagny Limited**
  **Tokyo 140-8710 (JP)**
- **SHIMOZATO, Takaichi**
  **c/o Sankyo Compagny Limited**
  **Tokyo 140-8710 (JP)**
- **KAGARI, Takashi**
  **c/o Sankyo Compagny Limited**
  **Tokyo 140-8710 (JP)**
- **DOI, Hiromi**
  **c/o Sankyo Compagny Limited**
  **Tokyo 140-8710 (JP)**

(74) Representative: **Wallace, Sheila Jane et al**
**Marks & Clerk LLP**
**90 Long Acre**
**London**
**WC2E 9RA (GB)**

(56) References cited:
**EP-A1- 1 772 145** **EP-A1- 1 915 994**
**WO-A1-96/06068** **WO-A1-98/45249**
**WO-A1-2004/024673** **WO-A1-2005/105146**
**JP-A- 2003 267 950**

- **LEUNG B.P. ET AL: 'A Novel Anti-Inflammatory Role for Simvastatin in Inflammatory Arthritis.' THE JOURNAL OF IMMUNOLOGY. vol. 170, no. 3, 2003, pages 1524 - 1530, XP002991714**
- **ITO A. ET AL: 'Long-term administration of atorvastatin, an HMG-CoA reductase inhibitor has beneficial effects on kidneys in autoimmune desease mice model.' NAGOYA MEDICAL JOURNAL. vol. 46, no. 1-2, 2003, pages 111 - 122, XP002991715**
- **ANDRZEJ G. ET AL: 'Statins as immunomodulatory drugs.' ADVANCES IN EXPERIMENTAL MEDICINE AND BIOLOGY. vol. 495, 2001, pages 285 - 288, XP002991716**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Remarks:

The file contains technical information submitted after the application was filed and not included in this specification

## Description

[Technical Field of the Invention]

The present invention relates to an excellent medicinal composition as a prophylactic or therapeutic agent for immune-related diseases such as reject reactions in organ or skin transplantation, comprising a 3-hydroxy-3-methylglutaryl coenzyme A reductase (hereinafter called as HMG-CoA reductase) inhibitor and one or more than one compound selected from amino alcohol derivatives having immunosuppressive activity, pharmacologically acceptable salts thereof, and pharmacological esters thereof as active ingredients.

[Background of the Invention]

In the past, anti-inflammatory agents such as steroids have been used for inflammatory reactions arising from abnormal immune response in the treatment of immune-related diseases such as rheumatoid arthritis and other autoimmune diseases. These are, however, a symptomatic therapy, but not a fundamental remedy. In addition, it has been reported that immune system abnormalities are also involved in the development of diabetes and nephritis (for example, see Non-patent literature 1 and Non-patent literature 2). Nevertheless, such medicaments to solve these abnormalities have never been developed.

Cyclosporin A (CsA), tacrolimus (TRL), and the like, which are previously existing immunosuppressants, are very important for preventing reject reactions in organ or skin transplantation, and for treatment or prophylaxis of various autoimmune diseases. However, these immunosuppressants are known to exert toxicities in the kidney and liver. To mitigate these side effects, concomitant treatment with immunosuppressants plus steroids is widely used. Nevertheless, significant immunosuppressive effects without adverse events are not always obtained.

In addition, although research and development of amino alcohol derivatives as immunosuppressants have recently been reported, these agents have not been widely used so far in clinical practice.

As the amino alcohol derivatives, for example, the following compounds are know:

(1) Compounds having the general formula (a) (for example, see Patent literature 1)

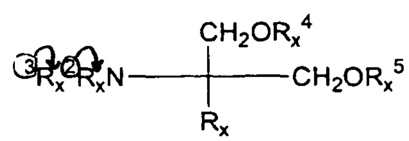

(a)

{in the above compounds (a),

$R_x$ represents a straight or branched carbon chain which may optionally be substituted with substituent (s) [said chain may contain a double bond, a triple bond, an oxygen atom, a sulfur atom, a group of formula -N($R_x^6$) - (wherein $R_x^6$ represents a hydrogen atom), an arylene group which may optionally be substituted with substituent(s), or a heteroarylene group which may optionally be substituted with substituent(s), and may contain, at the end of said chain, an aryl group which may optionally be substituted with substituent(s), a cycloalkyl group which may optionally be substituted with substituent(s), or a heteroaryl group which may optionally be substituted with substituent(s)], and $R_x^2$, $R_x^3$, $R_x^4$ and $R_x^5$ are the same or different and each represents a hydrogen atom or an alkyl group}.

(2) Compounds having the general formula (b) (for example, see Patent literature 2)

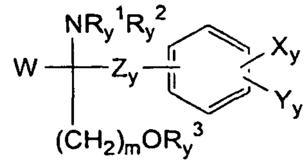

(b)

[in the above compounds (b),

$R_y^1$,$R_y^2$, and $R_y^3$ each represent a hydrogen atom or the like; W represents a hydrogen atom, an alkyl group or the like; $Z_y$ represents a single bond or an alkylene group; $X_y$ represents a hydrogen atom or an alkoxy group, and $Y_y$

represents a hydrogen atom, an alkyl group, an alkoxy group, an acyl group, an acyloxy group, an amino group, an acylamino group or the like].

(3) Compounds having the general formula (c) (for example, see Patent literature 3)

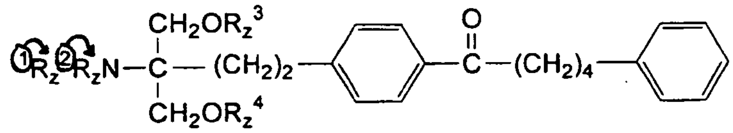

(c)

[in the above compounds (c),

$R_z^1$, $R_z^2$, $R_z^3$, and $R_z^4$ are the same or different and each represents a hydrogen atom or an acyl group].

(4) Compounds having the general formula (d) (for example, see Patent literatures 4 and 5)

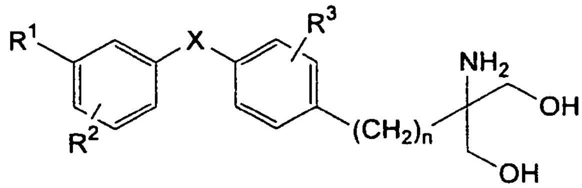

(d)

[in the above compounds (d),

$R^1$ represents a halogen atom, a trihalomethyl group, a hydroxyl group, a lower alkyl group having from 1 to 7 carbon atoms, a phenoxymethyl group, or the like; $R^2$ represents a hydrogen atom, a halogen atom, a trihalomethyl group or the like; $R^3$ represents a hydrogen atom, a halogen atom, a trihalomethyl group or the like; X represents an oxygen atom, a sulfur atom, a SO group or a $SO_2$ group; and n represents an integer of from 1 to 4].

On the other hand, this applicant disclosed the compounds having the following general formula (e) according to Japanese Patent Official Gazette 2002-167382 (Patent literature 6).

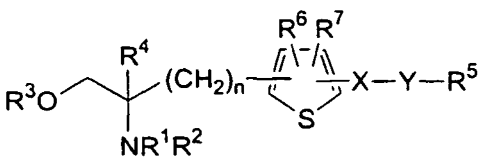

(e)

[wherein, $R^1$ and $R^2$ represent a hydrogen atom, or a protective group for the amino group; $R^3$ represents a hydrogen atom, or a protective group for the hydroxyl group; $R^4$ represents a lower alkyl group; n represents an integer of from 1 to 6; X represents an ethylene group or the like; Y represents a $C_1$-$C_{10}$ alkylene group or the like; $R^5$ represents an aryl group, an aryl group substituted with substituent(s), or the like; $R^6$ and $R^7$ represent a hydrogen atom or the like; when $R^5$ represents a hydrogen atom, however, Y represents a group other than a single bond and a straight chained $C_1$-$C_{10}$ alkylene group].

Additionally, this applicant disclosed the compounds having the following general formula (f) according to Japanese Patent Official Gazette 2003-267950 (Patent literature 7).

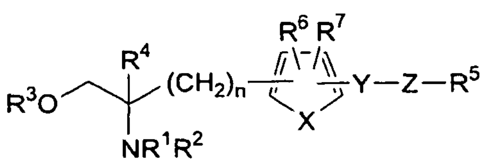

(f)

[wherein, $R^1$ and $R^2$ are the same or different and each represents a hydrogen atom, a protective group for the amino group or the like; $R^3$ represents a hydrogen atom, a protective group for the hydroxyl group or the like; $R^4$ represents a lower alkyl group; n represents an integer of from 1 to 6; X represents an oxygen atom or a nitrogen atom which is not substituted or is substituted with a lower alkyl group; Y represents an ethylene group or the like; Z represents an alkylene group having from 1 to 10 carbon atoms or the like; $R^5$ represents an aryl group, an aryl group which is substituted with substituent(s), or the like; $R^6$ and $R^7$ represent a hydrogen atom or the like; when $R^5$ represents a hydrogen atom, however, Z represents a group other than a single bond and a straight chained alkylene group having from 1 to 10 carbon atoms].

Additionally, it has become widely known that the amino alcohol derivatives described above exert activities by phosphorylation of the hydroxyl group in the molecule of said derivatives in vivo.

On the other hand, an HMG-CoA reductase inhibitor was recently reported to exhibit therapeutic effects against rheumatic arthritis (see Non-patent literature 3 and Non-patent literature 4). However, since the efficacy was reported in studies carried out in limited cases, it is still unclear whether HMG-CoA reductase inhibitors are generally effective.

In light of this background, it is desired to discover medicinal compositions exerting excellent immunosuppressive activity with low toxicity.

[Non-patent literature 1] Kidney International, Vol. 51, 94 (1997)
[Non-patent literature 2] Journal of Immunology, vol. 157, 4691 (1996)
[Non-patent literature 3] Journal of Rheumatology, 2002 Sep; 29(9): 2024-6.
[Non-patent literature 4] Lupus. 2003; 12(8): 607-11.
[Patent literature 1] WO94/08943 (EP627406)
[Patent literature 2] WO96/06068
[Patent literature 3] WO98/45249
[Patent literature 4] WO03/029184
[Patent literature 5] WO03/029205
[Patent literature 6] Japanese Patent Publication (Kokai) Number 2002-167382
[Patent literature 7] Japanese Patent Publication (Kokai) Number 2003-267950

[Object of the Invention]

The object of the present invention is to provide excellent medicinal compositions as a prophylactic or a therapeutic agent for immune-related diseases such as reject reactions caused by organ or skin transplantation, and autoimmune diseases such as rheumatic arthritis, psoriasis, inflammatory bowel disease, multiple sclerosis, and other autoimmune diseases.

The present inventors have eagerly studied to develop a medicinal composition having excellent immunosuppressive activity and found a medicinal composition of the present invention having excellent immunosuppressive effects and low toxicity, and that the medicinal composition augmented a pharmacological activity of each immunosuppressant contained in the medicinal composition described above, and lowered side effects of each immunosuppressant and that the medicinal composition is useful as a prophylactic or therapeutic agent for autoimmune diseases such as systemic lupus erythematosus, rheumatoid arthritis, polymyositis, dermatomyositis, scleroderma, Behcet's syndrome, Crohn's disease, ulcerative colitis, autoimmune hepatitis, aplastic anemia, idiopathic thrombocytopenic purpura, autoimmune hemolytic anemia, multiple sclerosis, autoimmune bullosis, psoriasis vulgaris, vasculitis syndrome, Wegener's granuloma, uveitis, idiopathic interstitial pneumonia, Goodpasture's syndrome, sarcoidosis, allergic granulomatous angitis, bronchial asthma, myocarditis, cardiomyopathy, aortitis syndrome, postmyocardial infarction syndrome, primary pulmonary hypertension, minimal change nephrotic syndrome, membranous nephropathy, membranoproliferative glomerulonephritis, focal glomerular sclerosis, crescentic glomerulonephritis, myasthenia gravis, inflammatory neuropathy, atopic dermatitis, chronic actinic dermatitis, acute polyarthritis, Sydenham's chorea, systemic sclerosis, adult-onset type diabetes mellitus, insulin dependent diabetes mellitus, juvenile diabetes mellitus, atherosclerosis, glomerular nephritis, tubulointerstitial nephritis, primary biliary cirrhosis, primary sclerosing cholangitis, fulminant hepatitis, viral hepatitis, GVHD, reject reactions caused by transplantation of various organs, contact dermatitis, and sepsis, or other immune-related diseases, and completed the present invention.

[Measures to achieve the object]

(1) a medicinal composition of the present invention is a medicinal composition comprising at least one agent selected from the group consisting of HMG-CoA reductase inhibitors and at least one compound selected from the

group consisting of amino alcohol derivatives having the general formula (I), pharmacologically acceptable salts thereof, and pharmacological esters thereof:

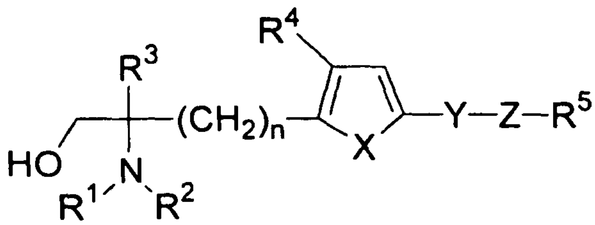

(I)

[wherein, $R^1$ and $R^2$ are the same or different and each represents a hydrogen atom, or a lower alkyl group,
$R^3$ represents a lower alkyl group or a hydroxymethyl group,
$R^4$ represents a hydrogen atom, a lower alkyl group, a lower alkoxy group or a halogen atom,
$R^5$ represents a hydrogen atom, a halogen atom, a cyclohexyl group, a phenyl group, or a phenyl group substituted with from 1 to 3 substituents selected from Substituent group a,
X represents a methylated nitrogen atom,
Y represents a single bond, an oxygen atom, a sulfur atom or a carbonyl group,
Z represents a single bond, an alkylene group having from 1 to 8 carbon atoms, or an alkylene group having from 1 to 8 carbon atoms which is substituted with from 2 to 8 fluorine atoms,
n represents an integer of 2 or 3, and
Substituent group a represents the group consisting of a halogen atom, a cyano group, a lower alkyl group, a lower cycloalkyl group, a lower alkoxy group, a trifluoromethyl group, a phenyl group, and a benzyloxy group.
the "lower alkyl group" is a methyl, ethyl, propyl, isopropyl, butyl, isobutyl, s-butyl, t-butyl, pentyl or hexyl group,
the "lower alkoxy group" is a methoxy, ethoxy, propoxy, isopropoxy, butoxy, isobutoxy, s-butoxy, t-butoxy, pentoxy or hexyloxy group, and
the "lower cycloalkyl group" is a cyclopropyl, cyclobutyl, cyclopentyl or cyclohexyl group.

Of the medicinal compositions according to (1) described above, preferred medicinal compositions are as follows:

(2) a medicinal composition according to (1) described above, wherein an amino alcohol derivative having the general formula (I) is a compound having the general formula (Ia) shown below,

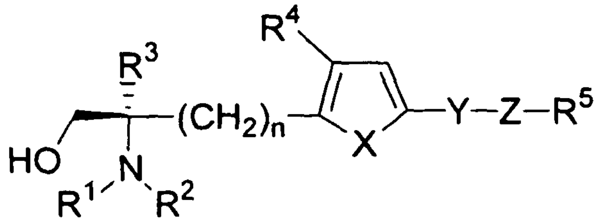

(Ia)

(3) a medicinal composition according to (1) or (2) described above, wherein the HMG-CoA reductase inhibitor is at least one agent selected from the group consisting of pravastatin, lovastatin, simvastatin, fluvastatin, atorvastatin, rosuvastatin and pitavastatin,
(4) a medicinal composition according to (1) or (2) described above, wherein the HMG-CoA reductase inhibitor is at least one agent selected from the group consisting of pravastatin, simvastatin and atorvastatin,
(5) a medicinal composition according to (1) or (2) described above, wherein the HMG-CoA reductase inhibitor is pravastatin,
(6) medicinal composition according to (1) or (2) described above, wherein the HMG-CoA reductase inhibitor is atorvastatin,
(7) a medicinal composition according to any one selected from (1) to (6) described above, which comprises an amino alcohol derivative having the general formula (I) wherein $R^1$ and $R^2$ are a hydrogen atom,

(8) a medicinal composition according to any one selected from (1) to (7) described above, which comprises an amino alcohol derivative having the general formula (I) wherein $R^3$ is a methyl, ethyl, or hydroxymethyl group,
(9) a medicinal composition according to any one selected from (1) to (7) described above, which comprises an amino alcohol derivative having the general formula (I) wherein $R^3$ is a methyl group or an ethyl group,
(10) a medicinal composition according to any one selected from (1) to (7) described above, which comprises an amino alcohol derivative having the general formula (I) wherein $R^3$ is a hydroxymethyl group,
(11) a medicinal composition according to any one selected from (1) to (7) described above, which comprises an amino alcohol derivative having the general formula (I) wherein $R^3$ is a methyl group,
(12) a medicinal composition according to any one selected from (1) to (11) described above, which comprises an amino alcohol derivative having the general formula (I) wherein $R^4$ is a hydrogen atom,
(13) a medicinal composition according to any one selected from (1) to (11) described above, which comprises an amino alcohol derivative having the general formula (I) wherein $R^4$ is a chlorine atom,
(14) a medicinal composition according to any one selected from (1) to (13) described above, which comprises an amino alcohol derivative having the general formula (I) wherein $R^5$ is a phenyl group or a phenyl group substituted with from 1 to 3 substituents selected from Substituent group a,
(15) a medicinal composition according to any one selected from (1) to (13) described above, which comprises an amino alcohol derivative having the general formula (I) wherein $R^5$ is a phenyl group substituted with from 1 to 3 substituents selected from the group consisting of a fluorine atom, a cyano group, a methyl group, a methoxy group, an ethoxy group and a phenyl group,
(16) a medicinal composition according to any one selected from (1) to (13) described above, which comprises an amino alcohol derivative having the general formula (I) wherein $R^5$ is a phenyl group substituted with a substituent selected from the group consisting of a fluorine atom, a cyano group, a methyl group, a methoxy group, an ethoxy group and a phenyl group,
(17) a medicinal composition according to any one selected from (1) to (13) described above, which comprises an amino alcohol derivative having the general formula (I) wherein $R^5$ is a phenyl group of which the para position is substituted with a substituent selected from the group consisting of a fluorine atom, a cyano group, a methyl group, a methoxy group, an ethoxy group and a phenyl group,
(18) a medicinal composition according to any one selected from (1) to (13) described above, which comprises an amino alcohol derivative having the general formula (I) wherein $R^5$ is a phenyl group substituted with a trifluoromethyl group or a benzyloxy group,
(19) a medicinal composition according to any one selected from (1) to (13) described above, which comprises an amino alcohol derivative having the general formula (I) wherein $R^5$ is a phenyl group of which the meta position is substituted with a trifluoromethyl group or a benzyloxy group,
(20) a medicinal composition according to any one selected from (1) to (13) described above, which comprises an amino alcohol derivative having the general formula (I) wherein $R^5$ is a phenyl group substituted with from 1 to 3 substituents selected from the group consisting of a lower alkyl group and a lower alkoxy group,
(21) a medicinal composition according to any one selected from (1) to (13) described above, which comprises an amino alcohol derivative having the general formula (I) wherein $R^5$ is a phenyl group substituted with from 1 to 3 substituents selected from the group consisting of a methyl group and a methoxy group,
(22) a medicinal composition according to any one selected from (1) to (13) described above, which comprises an amino alcohol derivative having the general formula (I) wherein $R^5$ is a phenyl group,
(23) a medicinal composition according to any one selected from (1) to (13) described above, which comprises an amino alcohol derivative having the general formula (I) wherein $R^5$ is a hydrogen atom,
(24) a medicinal composition according to any one selected from (1) to (23) described above, which comprises an amino alcohol derivative having the general formula (I) wherein Y represents a single bond,
(25) a medicinal composition according to any one selected from (1) to (23) described above, which comprises an amino alcohol derivative having the general formula (I) wherein Y represents an oxygen atom,
(26) a medicinal composition according to any one selected from (1) to (23) described above, which comprises an amino alcohol derivative having the general formula (I) wherein Y represents a sulfur atom,
(27) a medicinal composition according to any one selected from (1) to (23) described above, which comprises an amino alcohol derivative having the general formula (I) wherein Y represents a carbonyl group,
(28) a medicinal composition according to any one selected from (1) to (27) described above, which comprises an amino alcohol derivative having the general formula (I) wherein Z represents a single bond or an alkylene group having from 1 to 8 carbon atoms,
(29) a medicinal composition according to any one selected from (1) to (27) described above, which comprises an amino alcohol derivative having the general formula (I) wherein Z represents a single bond,
(30) a medicinal composition according to any one selected from (1) to (27) described above, which comprises an amino alcohol derivative having the general formula (I) wherein Z represents an alkylene group having from 1 to 8

carbon atoms,
(31) a medicinal composition according to any one selected from (1) to (27) described above, which comprises an amino alcohol derivative having the general formula (I) wherein Z represents an alkylene group having 8 carbon atoms,
(32) a medicinal composition according to any one selected from (1) to (27) described above, which comprises an amino alcohol derivative having the general formula (I) wherein Z represents an alkylene group having from 2 to 5 carbon atoms,
(33) a medicinal composition according to any one selected from (1) to (27) described above, which comprises an amino alcohol derivative having the general formula (I) wherein Z represents an alkylene group having 3 carbon atoms,
(34) a medicinal composition according to any one selected from (1) to (33) described above, which comprises an amino alcohol derivative having the general formula (I) wherein n represents an integer of 2,
(35) a medicinal composition according to any one selected from (1) to (33) described above, which comprises an amino alcohol derivative having the general formula (I) wherein n represents an integer of 3,
(36) a medicinal composition according to any one selected from (1) to (6) described above, wherein an amino alcohol derivative having the general formula (I) is the following compound:

2-amino-2-methyl-4-{1-methyl-5-[4-(2-methylphenyl)butanoyl] pyrrol-2-yl}butan-1-ol,
2-amino-2-methyl-4-{1-methyl-5-[4-(3-methylphenyl)butanoyl] pyrrol-2-yl}butan-1-ol,
2-amino-2-methyl-4-{1-methyl-5-[4-(4-methylphenyl)butanoyl] pyrrol-2-yl}butan-1-ol,
2-amino-2-methyl-4-{1-methyl-5-[4-(2,3-dimethylphenyl)butanoyl] pyrrol-2-yl}butan-1-ol,
2-amino-2-methyl-4-{1-methyl-5-[4-(2,4-dimethylphenyl)butanoyl] pyrrol-2-yl}butan-1-ol,
2-amino-2-methyl-4-{1-methyl-5-[4-(2,5-dimethylphenyl)butanoyl] pyrrol-2-yl}butan-1-ol,
2-amino-2-methyl-4-{1-methyl-5-[4-(3,4-dimethylphenyl)butanoyl] pyrrol-2-yl}butan-1-ol,
2-amino-2-methyl-4-{1-methyl-5-[4-(3,5-dimethylphenyl)butanoyl] pyrrol-2-yl}butan-1-ol,
2-amino-2-methyl-4-{1-methyl-5-[4-(4-ethylphenyl)butanoyl] pyrrol-2-yl}butan-1-ol,
2-amino-2-methyl-4-{1-methyl-5-[4-(4-isopropylphenyl)butanoyl] pyrrol-2-yl}butan-1-ol,
2-amino-2-methyl-4-{1-methyl-5-[4-(4-cyclopropylphenyl)butanoyl) pyrrol-2-yl}butan-1-ol,
2-amino-2-methyl-4-{1-methyl-5-[4-(4-t-butylphenyl)butanoyl] pyrrol-2-yl}butan-1-ol,
2-amino-2-methyl-4-{1-methyl-5-[4-(4-methoxyphenyl)butanoyl] pyrrol-2-yl}butan-1-ol,
2-amino-2-methyl-4-{1-methyl-5-[4-(3,4-dimethoxyphenyl)butanoyl] pyrrol-2-yl}butan-1-ol,
2-amino-2-methyl-4-{1-methyl-5-[4-(3-methyl-4-methoxyphenyl) butanoyl]pyrrol-2-yl}butan-1-ol,
2-amino-2-methyl-4-{1-methyl-5-[4-(3-methoxy-4-methylphenyl) butanoyl]pyrrol-2-yl}butan-1-ol,
2-amino-2-methyl-4-{1-methyl-5-[4-(4-fluorophenyl)butanoyl] pyrrol-2-yl}butan-l-ol,
2-amino-2-methyl-4-{1-methyl-5-[4-(4-chlorophenyl)butanoyl] pyrrol-2-yl}butan-1-ol,
2-amino-2-methyl-4-{1-methyl-5-(4-(4-trifluoromethylphenyl) butanoyl]pyrrol-2-yl}butan-1-ol,
2-amino-2-methyl-4-{1-methyl-5-[4-(4-cyanophenyl)butanoyl] pyrrol-2-yl}butan-1-ol,
2-amino-2-ethyl-4-{1-methyl-5-[4-(2-methylphenyl)butanoyl] pyrrol-2-yl}butan-1-ol,
2-amino-2-ethyl-4-{1-methyl-5-[4-(3-methylphenyl)butanoyl] pyrrol-2-yl}butan-1-ol,
2-amino-2-ethyl-4-{1-methyl-5-[4-(4-methylphenyl)butanoyl] pyrrol-2-yl}butan-1-ol,
2-amino-2-ethyl-4-{1-methyl-5-[4-(2,3-dimethylphenyl)butanoyl] pyrrol-2-yl}butan-1-ol,
2-amino-2-ethyl-4-{1-methyl-5-[4-(2,4-dimethylphenyl)butanoyl] pyrrol-2-yl}butan-1-ol,
2-amino-2-ethyl-4-{1-methyl-5-[4-(2,5-dimethylphenyl)butanoyl] pyrrol-2-yl}butan-1-ol,
2-amino-2-ethyl-4-{1-methyl-5-[4-(3,4-dimethylphenyl)butanoyl] pyrrol-2-yl}butan-1-ol,
2-amino-2-ethyl-4-{1-methyl-5-[4-(3,5-dimethylphenyl)butanoyl] pyrrol-2-yl}butan-1-ol,
2-amino-2-ethyl-4-{1-methyl-5-[4-(4-ethylphenyl)butanoyl] pyrrol-2-yl}butan-1-ol,
2-amino-2-ethyl-4-{1-methyl-5-[4-(4-isopropylphenyl)butanoyl] pyrrol-2-yl}butan-1-ol,
2-amino-2-ethyl-4-{1-methyl-5-[4-(4-cyclopropylphenyl)butanoyl] pyrrol-2-yl}butan-1-ol,
2-amino-2-ethyl-4-{1-methyl-5-[4-(4-t-butylphenyl)butanoyl] pyrrol-2-yl}butan-1-ol,
2-amino-2-ethyl-4-{1-methyl-5-[4-(4-methoxyphenyl)butanoyl] pyrrol-2-yl}butan-1-ol,
2-amino-2-ethyl-4-{1-methyl-5-[4-(3,4-dimethoxyphenyl)butanoyl] pyrrol-2-yl}butan-1-ol,
2-amino-2-ethyl-4-{1-methyl-5-[4-(3-methyl-4-methoxyphenyl) butanoyl]pyrrol-2-yl}butan-1-ol,
2-amino-2-ethyl-4-{1-methyl-5-[4-(4-fluorophenyl)butanoyl] pyrrol-2-yl}butan-1-ol,
2-amino-2-ethyl-4-{1-methyl-5-[4-(4-chlorophenyl)butanoyl] pyrrol-2-yl}butan-1-ol,
2-amino-2-ethyl-4-{1-methyl-5-[4-(4-trifluoromethylphenyl) butanoyl]pyrrol-2-yl}butan-l-ol,
2-amino-2-ethyl-4-{1-methyl-5-[4-(4-cyanophenyl)butanoyl] pyrrol-2-yl}butan-1-ol,
2-amino-2-methyl-4-{1-methyl-5-[5-(2-methylphenyl)pentanoyl] pyrrol-2-yl}butan-1-ol,
2-amino-2-methyl-4-{1-methyl-5-[5-(3-methylphenyl)pentanoyl] pyrrol-2-yl}butan-1-ol,

2-amino-2-methyl-4-{1-methyl-5-[5-(4-methylphenyl)pentanoyl] pyrrol-2-yl}butan-1-ol,
2-amino-2-methyl-4-{1-methyl-5-[5-(2,3-dimethylphenyl)pentanoyl] pyrrol-2-yl}butan-1-ol,
2-amino-2-methyl-4-{1-methyl-5-[5-(2,4-dimethylphenyl)pentanoyl] pyrrol-2-yl}butan-1-ol,
2-amino-2-methyl-4-{1-methyl-5-[5-(2,5-dimethylphenyl)pentanoyl] pyrrol-2-yl}butan-1-ol,
2-amino-2-methyl-4-{1-methyl-5-[5-(3,4-dimethylphenyl)pentanoyl] pyrrol-2-yl}butan-1-ol,
2-amino-2-methyl-4-{1-methyl-5-[5-(3,5-dimethylphenyl)pentanoyl] pyrrol-2-yl}butan-1-ol,
2-amino-2-methyl-4-{1-methyl-5-[5-(3-methyl-4-methoxyphenyl) pentanoyl]pyrrol-2-yl}butan-1-ol,
2-mino-2-methyl-4-{1-methyl-5-[5-(3-methoxy-4-methylphenyl) pentanoyl]pyrrol-2-yl}butan-1-ol, or
2-amino-2-methyl-4-{1-methyl-5-[5-(4-cyanophenyl)pentanoyl] pyrrol-2-yl}butan-1-ol,

(37) a medicinal composition according to any one selected from (1) to (6) described above, wherein an amino alcohol derivative having the general formula (I) is the following compound:

2-amino-2-methyl-4-{1-methyl-5-[4-(2-methylphenyl)butanoyl] pyrrol-2-yl}butan-1-ol,
2-amino-2-methyl-4-{1-methyl-5-[4-(3-methylphenyl)butanoyl] pyrrol-2-yl}butan-1-ol,
2-amino-2-methyl-4-{1-methyl-5-[4-(4-methylphenyl)butanoyl] pyrrol-2-yl}butan-1-ol,
2-amino-2-methyl-4-{1-methyl-5-[4-(2,3-dimethylphenyl)butanoyl] pyrrol-2-yl}butan-1-ol,
2-amino-2-methyl-4-{1-methyl-5-[4-(2,4-dimethylphenyl)butanoyl] pyrrol-2-yl}butan-1-ol,
2-amino-2-methyl-4-{1-methyl-5-[4-(2,5-dimethylphenyl)butanoyl] pyrrol-2-yl}butan-1-ol,
2-amino-2-methyl-4-{1-methyl-5-[4-(3,4-dimethylphenyl)butanoyl] pyrrol-2-yl}butan-1-ol,
2-amino-2-methyl-4-{1-methyl-5-[4-(3,5-dimethylphenyl)butanoyl] pyrrol-2-yl}butan-1-ol,
2-amino-2-methyl-4-{1-methyl-5-[4-(3-methyl-4-methoxyphenyl) butanoyl]pyrrol-2-yl}butan-1-ol,
2-amino-2-methyl-4-{1-methyl-5-[4-(3-methoxy-4-methylphenyl) butanoyl]pyrrol-2-yl}butan-1-ol,
2-amino-2-methyl-4-{1-methyl-5-[4-(4-cyanophenyl)butanoyl] pyrrol-2-yl}butan-1-ol,
2-amino-2-methyl-4-{1-methyl-5-[5-(2-methylphenyl)pentanoyl] pyrrol-2-yl}butan-1-ol,
2-amino-2-methyl-4-{1-methyl-5-[5-(3-methylphenyl)pentanoyl] pyrrol-2-yl}butan-1-ol,
2-amino-2-methyl-4-{1-methyl-5-[5-(4-methylphenyl)pentanoyl] pyrrol-2-yl}butan-1-ol,
2-amino-2-methyl-4-{1-methyl-5-[5-(2,3-dimethylphenyl)pentanoyl] pyrrol-2-yl}butan-1-ol,
2-amino-2-methyl-4-{1-methyl-5-[5-(2,4-dimethylphenyl)pentanoyl] pyrrol-2-yl}butan-1-ol,
2-amino-2-methyl-4-{1-methyl-5-[5-(2,5-dimethylphenyl)pentanoyl] pyrrol-2-yl}butan-1-ol,
2-amino-2-methyl-4-{1-methyl-5-[5-(3,4-dimethylphenyl)pentanoyl] pyrrol-2-yl}butan-1-ol,
2-amino-2-methyl-4-{1-methyl-5-[5-(3,5-dimethylphenyl)pentanoyl] pyrrol-2-yl}butan-1-ol,
2-amino-2-methyl-4-{1-methyl-5-[5-(3-methyl-4-methoxyphenyl) pentanoyl]pyrrol-2-yl}butan-1-ol,
2-amino-2-methyl-4-{1-methyl-5-[5-(3-methoxy-4-methylphenyl) pentanoyl]pyrrol-2-yl}butan-1-ol, or
2-amino-2-methyl-4-{1-methyl-5-[5-(4-cyanophenyl)pentanoyl] pyrrol-2-yl}butan-1-ol,

Additionally, the present invention provides

(38) a medicinal composition according to any one selected from (1) to (37), in the form of a combination drug for use in the treatment or prophylaxis of an autoimmune disease,
(39) a medicinal composition according to any one selected from (1) to (37), in the form of a kit for use in the treatment or prophylaxis of an autoimmune disease, wherein said kit comprises a pharmaceutical preparation of said at least one HMG-CoA reductase inhibitor agent and a pharmaceutical preparation of said at least one amino alcohol derivative compound,
(40) use of a medicinal composition according to any one selected from (1) to (37) for the manufacture of a medicament for prophylaxis or treatment of an autoimmune disease,
(41) use of at least one agent selected from the group of HMG-CoA reductase inhibitors and at least one compound selected from the group consisting of amino alcohol derivatives having the general formula (I), pharmacologically acceptable salts thereof and pharmacologically acceptable esters thereof as defined in any one selected from (1) to (37) for the manufacture of a medicament for administering said at least one agent and said at least one compound simultaneously, sequentially or separately on different occasions for prophylaxis or treatment of an autoimmune disease, and
(42) a method of preparing a medicinal composition according to any one selected from (1) to (37), which comprises mixing at least one agent selected from the group of HMG-CoA reductase inhibitors and at least one compound selected from the group consisting of amino alcohol derivatives having the general formula (I), pharmaceutically acceptable salts thereof and pharmacologically acceptable esters thereof.

The HMG-CoA reductase inhibitors that are one of the active ingredients of the medicinal composition of the

present invention encompass all natural materials originated from organisms, semi-synthesized compounds derived from said natural materials and wholly synthesized compounds thereof, and are, for example, (+)-(3R,5R)-3,5-dihydroxy-7-[(1S,2S,6S,8S,8aR)-6-hydroxy-2-methyl-8-[(S)-2-methyl-butyryloxy]-1,2,6,7,8,8a-hexahydro-1-naphthyl]heptanoic acid (Pravastatin, Japanese Patent Publication (Kokai) Sho 57-2240 (USP 4346227)), (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl] ethyl]-1-naphthyl (S)-2-methylbutyrate (Lovastatin, Japanese Patent Publication (Kokai) Sho 57-163374 (USP 4231938)), (+)-(1S,3R,7S,8S,8aR)-1,2,3,7,8,8a-hexahydro-3,7-dimethyl-8-[2-[(2R,4R)-tetrahydro-4-hydroxy-6-oxo-2H-pyran-2-yl]ethyl]-1-naphthyl 2,2-dimethylbutyrate (Simvastatin, Japanese Patent Publication (Kokai) Sho 56-122375 (USP 4444784)), (±)-(3R*,5S*,6E)-7-[3-(4-fluorophenyl)-1-(1-methylethyl)-1H-indol-2-yl]-3,5-dihydroxy-6-heptenoic acid (Fluvastatin, Japanese Patent Publication (Kokai) Sho 60-500015 (USP 4739073)), (3R,5S)-7-[2-(4-fluorophenyl)-5-(1-methylethyl)-3-phenyl-4-phenylaminocarbonyl-1H-pyrrol-1-yl]-3,5-dihydroxyheptenoic acid (Atorvastatin, Japanese Patent Publication (Kokai) Hei 3-58967 (USP 5273995)), (+)-(3R,5S)-7-[4-(4-fluorophenyl)-6-isopropyl-2-(N-methyl-N-methanesulfonylamino)-pyrimidin-5-yl]-3,5-dihydroxy-6 (E)-heptenoic acid (Rosuvastatin, Japanese Patent Publication (Kokai) Hei 5-178841 (USP 5260440)) or (E)-3,5-dihydroxy-7-[4'-(4''-fluorophenyl)-2'-cyclopropylquinolin-3'-yl]-6-heptenoic acid (Pitavastatin, Japanese Patent Publication (Kokai) Hei 1-279866 (USP 5854259 and USP 5856336)).

The HMG-CoA reductase inhibitor is preferably pravastatin, lovastatin, simvastatin, fluvastatin, atorvastatin, rosuvastatin or pitavastatin, more preferably pravastatin, simvastatin or atorvastatin, most preferably pravastatin or atorvastatin, and particularly preferably atorvastatin.

Planar chemical structures of representative HMG-CoA reductase inhibitors are shown below:

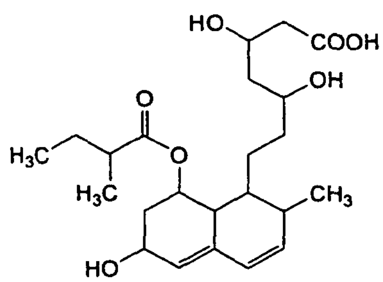

Pravastatin

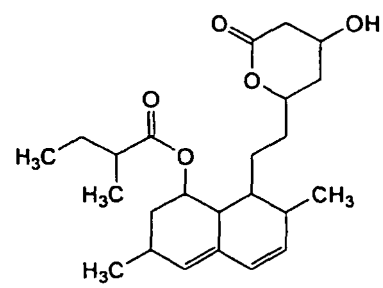

Lovastatin

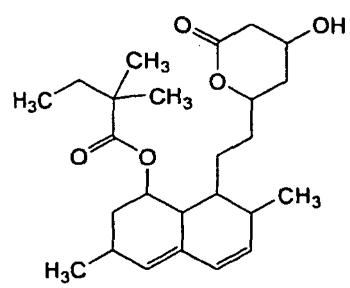

Simvastatin

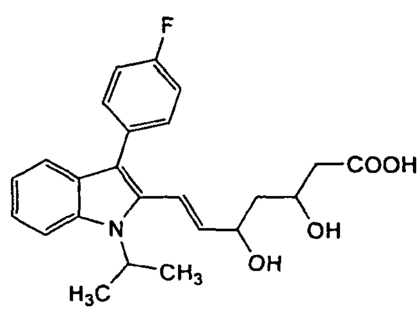

Fluvastatin

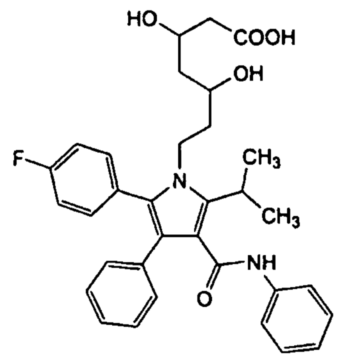

Atorvastatin

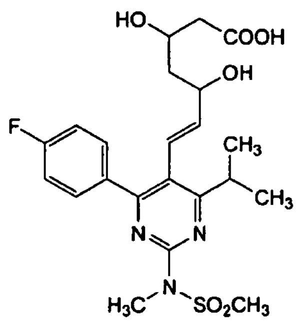

Rosuvastatin

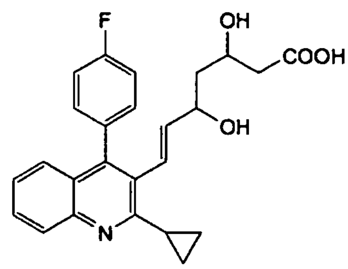

Pitavastatin

The amino alcohol derivatives, which are one of active ingredients of the medicinal composition of the present invention, have the following general formula (I).

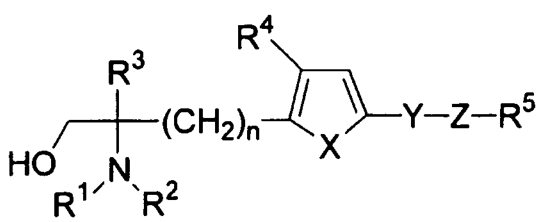

(I)

In the formula shown above, the "lower alkyl group" in the definition of $R^1$, $R^2$, $R^3$, $R^4$ and Substituent group a is a methyl, ethyl, propyl, isopropyl, butyl, isobutyl, s-butyl, t-butyl, pentyl or hexyl group, and is preferably a methyl, ethyl or propyl group, and more preferably a methyl or ethyl group.

In the formula shown above, the "halogen atom" in the definition of $R^4$, $R^5$ and Substituent group a is a fluorine atom, a chlorine atom, a bromine atom or an iodine atom, and preferably a fluorine atom or a chlorine atom.

In the formula shown above, the "lower alkoxy group" in the definition of $R^4$ and Substituent group a is a methoxy, ethoxy, propoxy, isopropoxy, butoxy, isobutoxy, s-butoxy, t-butoxy, pentoxy or hexyloxy group.

In the formula shown above, the "alkylene group having from 1 to 8 carbon atoms" in the definition of Z can be, for example, a methylene, ethylene, propylene, tetramethylene, pentamethylene, hexamethylene, heptamethylene, or octamethylene group, and is preferably an alkylene group having from 2 to 8 carbon atoms, and more preferably an ethylene, propylene, tetramethylene, heptamethylene, or octamethylene group.

In the formula shown above, the "alkylene group having from 1 to 8 carbon atoms which is substituted with from 2 to 8 fluorine atoms" in the definition of Z can be, for example, a difluoromethylene, 1,1-difluoroethylene, 1,1,2,2-tetrafluoroethylene, 1,1-difluoropropylene, 1,1,2,2-tetrafluoropropylene, 1,1-difluorotetramethylene, 1,1,2,2-tetrafluorotetramethylene, 1,1-difluoropentamethylene or 1,1,2,2-tetrafluoropentamethylene group, and is preferably a 1,1-difluoropropylene, 1,1,2,2-tetrafluoropropylene, 1,1-difluorotetramethylene, 1,1,2,2-tetrafluorotetramethylene, 1,1-difluoropentamethylene or 1,1,2,2-tetrafluoropentamethylene group.

In the formula shown above, the "cycloalkyl group" in the definition of Substituent group a is a cyclopropyl, cyclobutyl, cyclopentyl or cyclohexyl group, and is preferably a cyclohexyl group.

The "pharmacologically acceptable salt thereof" described hereinbefore means a salt which can be prepared by reacting the compounds with an acid, as the compounds having the general formula (I) described hereinbefore have an amino group as a basic group. Such salt can be, for example, an inorganic acid salt, including a hydrohalide such as hydrofluoride, hydrochloride, hydrobromide, or hydroiodide, a nitrate, a perchlorate, a sulfate, a phosphate or the like; an organic acid salt, including a lower alkanesulfonate such as methanesulfonate, trifluoromethanesulfonate, or ethanesulfonate, an arylsulfonate such as benzenesulfonate or p-toluenesulfonate, an acetate, a malate, a fumarate, a succinate, a citrate, an ascorbate, a tartrate, an oxalate, a maleate, or the like; or an amino acid salt such as glycine salt, lysine salt, arginine salt, ornithine salt, glutamic acid salt, or aspartic acid salt, and is preferably hydrochloride, an acetate, a fumarate, a succinate or a maleate.

The "pharmacologically acceptable ester thereof" described hereinbefore means an ester of a compound having the general formula (I) described hereinbefore which has a hydroxyl group capable being esterified, and each ester residual group belongs to a "general protective group for the hydroxyl group".

The "general protective group for the hydroxyl group" means a "protective group in chemical reactions" which can be cleaved by a chemical process such as hydrogenolysis, hydrolysis, electrolysis or photolysis, and a "protective group which can be removed by a biological process such as hydrolysis in vivo".

Such the "protective group in chemical reactions" can be, for example, an "aliphatic acyl group", including an alkylcarbonyl group such as a formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, icosanoyl or henicosanoyl group, an alkylcarbonyl group substituted with a carboxyl group such as a succinoyl, glutaroyl or adipoyl group, a halogeno lower alkylcarbonyl group such as a chloroacetyl, dichloroacetyl, trichloroacetyl or trifluoroacetyl group, a lower alkylcarbonyl group substituted with lower alkoxy group (s) such as a methoxyacetyl group, and an unsaturated alkylcarbonyl group such as a (E)-2-methyl-2-butenoyl group; an "aromatic acyl group", including an arylcarbonyl group such as a benzoyl, α-naphthoyl or β-naphthoyl group, a halogeno arylcarbonyl group such as a 2-bromobenzoyl or 4-chlorobenzoyl group, an arylcarbonyl group substituted with lower alkyl groups such as a 2,4,6-trimethylbenzoyl or 4-toluoyl group, a lower alkoxylated arylcarbonyl group such as a 4-anisoyl group, an arylcarbonyl group substituted with carboxyl group(s) such as a 2-carboxybenzoyl, 3-carboxybenzoyl or 4-carboxybenzoyl group, a nitrated arylcarbonyl group such as a 4-nitrobenzoyl or 2-nitrobenzoyl group, an arylcarbonyl group substituted with lower alkoxycarbonyl group (s) such as a 2-(methoxycarbonyl)benzoyl group, and an arylcarbonyl group substituted with aryl group(s) such as a 4-phenylbenzoyl group; a "tetrahydropyranyl or tetrahydrothiopyranyl group" such as a tetrahydropyran-2-yl, 3-bromotetrahydropyran-2-yl, 4-methoxytetrahydropyran-4-yl, tetrahydrothiopyran-2-yl or 4-methoxytetrahydrothiopyran-4-yl group; a "tetrahydrofuranyl or tetrahydrothiofuranyl group" such as a tetrahydrofuran-2-yl or tetrahydrothiofuran-2-yl group; a "silyl group", including a tri-lower alkylsilyl group such as a trimethylsilyl, triethylsilyl, isopropyldimethylsilyl, t-butyldimethylsilyl, methyldiisopropylsilyl, methyldi-t-butylsilyl or triisopropylsilyl group and a tri-lower alkylsilyl group substituted with 1 or 2 aryl groups such as a diphenylmethylsilyl, diphenylbutylsilyl, diphenylisopropylsilyl or phenyldiisopropylsilyl group; an "alkoxymethyl group", including a lower alkoxymethyl group such as a methoxymethyl, 1,1-dimethyl-1-methoxymethyl, ethoxymethyl, propoxymethyl, isopropoxymethyl, butoxymethyl or t-butoxymethyl group, a lower alkoxymethyl group substituted with lower alkoxy group(s) such as a 2-methoxyethoxymethyl group, and a halogeno lower alkoxymethyl group such as a 2,2,2-trichloroethoxymethyl or bis(2-chloroethoxy)methyl group; a "substituted ethyl group", including a lower alkoxylated ethyl group such as a 1-ethoxyethyl or 1- (isopropoxy) ethyl group, and a halogenated ethyl group such as a 2,2,2-trichloroethyl group; an "aralkyl group", including a lower alkyl group substituted with from 1 to 3 aryl groups such as a benzyl, α-naphthylmethyl, β-naphthylmethyl, diphenylmethyl, triphenylmethyl, α-naphthyldiphenylmethyl or 9-anthrylmethyl group, and a lower alkyl group substituted with from 1 to 3 aryl groups, of which an aryl ring is substituted with lower alkyl group (s), lower alkoxy group(s), halogen atom(s) or cyano group(s), such as a 4-methylbenzyl, 2,4,6-trimethylbenzyl, 3,4,5-trimethylbenzyl, 4-methoxybenzyl, 4-methoxyphenyldiphenylmethyl, 2-nitrobenzyl, 4-nitrobenzyl, 4-chlorobenzyl, 4-bromobenzyl, 4-cyanobenzyl, methyl, or piperonyl group; an "alkoxycarbonyl group", including a lower alkoxycarbonyl group such as a methoxycarbonyl, ethoxycarbonyl, t-butoxycarbonyl or isobutoxycarbonyl group, and a lower alkoxycarbonyl group substituted with halogen atom(s) or tri-lower alkylsilyl group(s) such as a 2,2,2-trichloroethoxycarbonyl or 2-trimethylsilylethoxycarbonyl group; an "alkenyloxycarbonyl group" such as a vinyloxycarbonyl or allyloxycarbonyl group; an "aralkyloxycarbonyl group", of which an aryl ring may be substituted with 1 or 2 substituents selected from a lower alkoxy group or a nitro group, such as a benzyloxycarbonyl, 4-methoxybenzyloxycarbonyl, 3,4-dimethoxybenzyloxycarbonyl, 2-nitrobenzyloxycarbonyl, or 4-nitrobenzyloxycarbonyl group, and is preferably an aliphatic acyl group.

On the other hand, the "protective group which can be removed by a biological process such as hydrolysis in vivo" can be, for example, an acyloxyalkyl group such as an ethylcarbonyloxymethyl, pivaloyloxymethyl, dimethylaminoacetyloxymethyl, or 1-acetoxyethyl group; a 1-(alkoxycarbonyloxy)alkyl group such as a 1-(methoxycarbonyloxy)ethyl, 1-(ethoxycarbonyloxy)ethyl, ethoxycarbonyloxymethyl, 1-(isopropoxycarbonyloxy)ethyl, 1-(t-butoxycarbonyloxy)ethyl, 1-(ethoxycarbonyloxy)propyl, or 1-(cyclohexyloxycarbonyloxy)ethyl group; a phthalidyl group; a carbonyloxyalkyl group, for example, an oxodioxolenylmethyl group such as a 4-methyl-oxodioxolenylmethyl, 4-phenyl-oxodioxolenylmethyl, or oxodioxolenylmethyl group; the aliphatic acyl groups described hereinbefore; the aromatic acyl groups described hereinbefore; a residual group of a half ester of succinic acid; a residual group of an ester of phosphoric acid; a residual group of an ester formation of an amino acid; a carbamoyl group; and a carbamoyl group substituted with 1 or 2 lower alkyl groups. Whether a derivative prepared has such a group can be determined as follows. The derivative under investigation is intravenously administered to a test animal such as a rat or mouse and the body fluids of the test animal are thereafter studied. If the parent compound of said derivative or a pharmacologically acceptable salt of the parent compound is removed in said body fluid, said derivative under investigation is judged to have a protective group which can be deprotected by a biological process. Of these protective groups described above, a residual group of an ester of phosphoric acid is preferred.

The amino alcohol derivatives having the general formula (I) of the present invention, pharmacologically acceptable salts thereof and pharmacologically acceptable esters thereof have asymmetric carbon atoms in their structures and can exist as optical isomers. In the amino alcohol derivatives having the general formula (I), a single optical isomer and a mixture of optical isomers are represented by the single general formula (I). The present invention encompasses all the optical isomers and mixtures thereof in optional ratios. For example, in the amino alcohol derivatives having the general formula (I) of the present invention, the amino group is attached to an asymmetric carbon atom, and the particularly preferred absolute configuration at this asymmetric carbon is the R configuration.

When the amino alcohol derivatives having the general formula (I), pharmacologically acceptable salts thereof and pharmacologically acceptable esters thereof are allowed to stand so that they are exposed to the atmosphere or are recrystallized, they may absorb water or water may be attached to them to form a hydrate. Such hydrates are also encompassed as the pharmacologically acceptable salts of amino alcohol derivatives having the general formula (I) of the present invention.

Typical examples of compounds having the general formula (I) of the present invention are listed in the following Table 1, but the present invention should not be limited to these compounds.

The meaning of the abbreviations in the following Table is shown below.

Ac: acetyl group
tBu: t-butyl group
Et: ethyl group
EtO: ethoxy group
Me: methyl group
MeO: methoxy group
Ph: phenyl group
cPr: cyclopropyl group,
iPr: isopropyl group

(Table 1)

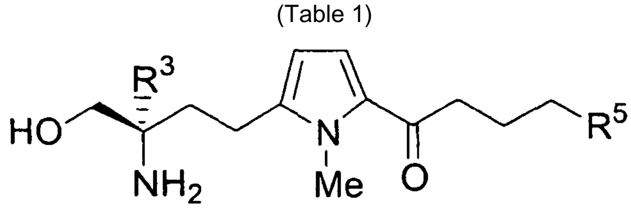

| Compound No. | $R^3$ | $R^5$ |
|---|---|---|
| 1 | Me | 2-F-Ph |
| 2 | Me | 3-F-Ph |
| 3 | Me | 4-F-Ph |
| 4 | Me | 2,3-di-F-Ph |
| 5 | Me | 2,4-di-F-Ph |

(continued)

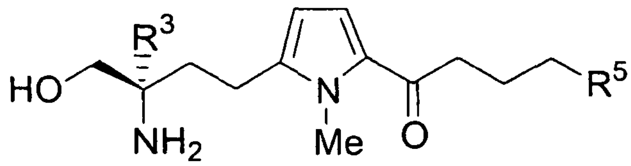

| Compound No. | $R^3$ | $R^5$ |
|---|---|---|
| 6 | Me | 2,5-di-F-Ph |
| 7 | Me | 3,4-di-F-Ph |
| 8 | Me | 3,5-di-F-Ph |
| 9 | Me | 2-Cl-Ph |
| 10 | Me | 3-Cl-Ph |
| 11 | Me | 4-Cl-Ph |
| 12 | Me | 2,3-di-Cl-Ph |
| 13 | Me | 2,4-di-Cl-Ph |
| 14 | Me | 2,5-di-Cl-Ph |
| 15 | Me | 3,4-di-Cl-Ph |
| 16 | Me | 3,5-di-Cl-Ph |
| 17 | Me | 2-Me-Ph |
| 18 | Me | 3-Me-Ph |
| 19 | Me | 4-Me-Ph |
| 20 | Me | 2,3-di-Me-Ph |
| 21 | Me | 2,4-di-Me-Ph |
| 22 | Me | 2,5-di-Me-Ph |
| 23 | Me | 3,4-di-Me-Ph |
| 24 | Me | 3,5-di-Me-Ph |
| 25 | Me | 2-Et-Ph |
| 26 | Me | 3-Et-Ph |
| 27 | Me | 4-Et-Ph |
| 28 | Me | 2-cPr-Ph |
| 29 | Me | 3-cPr-Ph |
| 30 | Me | 4-cPr-Ph |
| 31 | Me | 2-iPr-Ph |
| 32 | Me | 3-iPr-Ph |
| 33 | Me | 4-iPr-Ph |
| 34 | Me | 2-tBu-Ph |
| 35 | Me | 3-tBu-Ph |
| 36 | Me | 4-tBu-Ph |
| 40 | Me | 2-MeO-Ph |
| 41 | Me | 3-MeO-Ph |
| 42 | Me | 4-MeO-Ph |
| 43 | Me | 2,3-di-MeO-Ph |
| 44 | Me | 2,4-di-MeO-Ph |
| 45 | Me | 2,5-di-MeO-Ph |
| 46 | Me | 3,4-di-MeO-Ph |
| 47 | Me | 3,5-di-MeO-Ph |
| 48 | Me | 2-EtO-Ph |
| 49 | Me | 3-EtO-Ph |
| 50 | Me | 4-EtO-Ph |
| 51 | Me | 2-iPrO-Ph |

(continued)

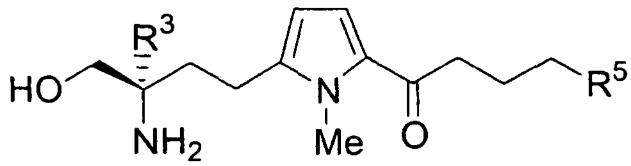

| Compound No. | $R^3$ | $R^5$ |
|---|---|---|
| 52 | Me | 3-iPrO-Ph |
| 53 | Me | 4-iPrO-Ph |
| 54 | Me | 2-Me-3-MeO-Ph |
| 55 | Me | 2-Me-4-MeO-Ph |
| 56 | Me | 2-Me-5-MeO-Ph |
| 57 | Me | 3-Me-4-MeO-Ph |
| 58 | Me | 3-Me-5-MeO-Ph |
| 59 | Me | 2-MeO-3-Me-Ph |
| 60 | Me | 2-MeO-4-Me-Ph |
| 61 | Me | 2-MeO-S-Me-Ph |
| 62 | Me | 3-MeO-4-Me-Ph |
| 63 | Me | 3-MeO-5-Me-Ph |
| 64 | Me | 2-$CF_3$-Ph |
| 65 | Me | 3-$CF_3$-Ph |
| 66 | Me | 4-$CF_3$-Ph |
| 67 | Me | 3,5-di-$CF_3$-Ph |
| 68 | Me | 2-Ac-Ph |
| 69 | Me | 3-Ac-Ph |
| 70 | Me | 4-Ac-Ph |
| 71 | Me | 2-CN-Ph |
| 72 | Me | 3-CN-Ph |
| 73 | Me | 4-CN-Ph |
| 74 | Et | 2-F-Ph |
| 75 | Et | 3-F-Ph |
| 76 | Et | 4-F-Ph |
| 77 | Et | 2,3-di-F-Ph |
| 78 | Et | 2,4-di-F-Ph |
| 79 | Et | 2,5-di-F-Ph |
| 80 | Et | 3,4-di-F-Ph |
| 81 | Et | 3,5-di-F-Ph |
| 82 | Et | 2-Cl-Ph |
| 83 | Et | 3-Cl-Ph |
| 84 | Et | 4-Cl-Ph |
| 85 | Et | 2,3-di-Cl-Ph |
| 86 | Et | 2,4-di-Cl-Ph |
| 87 | Et | 2,5-di-Cl-Ph |
| 88 | Et | 3,4-di-Cl-Ph |
| 89 | Et | 3,5-di-Cl-Ph |
| 90 | Et | 2-Me-Ph |
| 91 | Et | 3-Me-Ph |
| 92 | Et | 4-Me-Ph |
| 93 | Et | 2,3-di-Me-Ph |
| 94 | Et | 2,4-di-Me-Ph |

(continued)

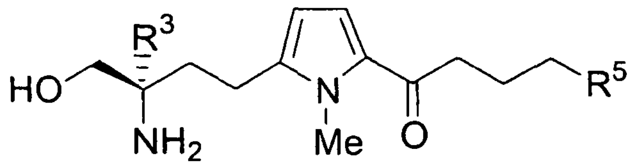

| Compound No. | $R^3$ | $R^5$ |
|---|---|---|
| 95 | Et | 2,5-di-Me-Ph |
| 96 | Et | 3,4-di-Me-Ph |
| 97 | Et | 3,5-di-Me-Ph |
| 98 | Et | 2-Et-Ph |
| 99 | Et | 3-Et-Ph |
| 100 | Et | 4-Et-Ph |
| 101 | Et | 2-cPr-Ph |
| 102 | Et | 3-cPr-Ph |
| 103 | Et | 4-cPr-Ph |
| 104 | Et | 2-iPr-Ph |
| 105 | Et | 3-iPr-Ph |
| 106 | Et | 4-iPr-Ph |
| 107 | Et | 2-tBu-Ph |
| 108 | Et | 3-tBu-Ph |
| 109 | Et | 4-tBu-Ph 4 tBu-Ph |
| 113 | Et | 2-MeO-Ph |
| 114 | Et | 3-MeO-Ph |
| 115 | Et | 4-MeO-Ph |
| 116 | Et | 2,3-di-MeO-Ph |
| 117 | Et | 2,4-di-MeO-Ph |
| 118 | Et | 2,5-di-MeO-Ph |
| 119 | Et | 3,4-di-MeO-Ph |
| 120 | Et | 3,5-di-MeO-Ph |
| 121 | Et | 2-EtO-Ph |
| 122 | Et | 3-EtO-Ph |
| 123 | Et | 4-EtO-Ph |
| 124 | Et | 2-iPrO-Ph |
| 125 | Et | 3-iPrO-Ph |
| 126 | Et | 4-iPrO-Ph |
| 127 | Et | 2-Me-3-MeO-Ph |
| 128 | Et | 2-Me-4-MeO-Ph |
| 129 | Et | 2-Me-5-MeO-Ph |
| 130 | Et | 3-Me-4-MeO-Ph |
| 131 | Et | 3-Me-5-MeO-Ph |
| 132 | Et | 2-MeO-3-Me-Ph |
| 133 | Et | 2-MeO-4-Me-Ph |
| 134 | Et | 2-MeO-5-Me-Ph |
| 135 | Et | 3-MeO-4-Me-Ph |
| 136 | Et | 3-MeO-5-Me-Ph |
| 137 | Et | 2-$CF_3$-Ph |
| 138 | Et | 3-$CF_3$-Ph |
| 139 | Et | 4-$CF_3$-Ph |
| 140 | Et | 3,5-di-$C_F$3-Ph |

(continued)

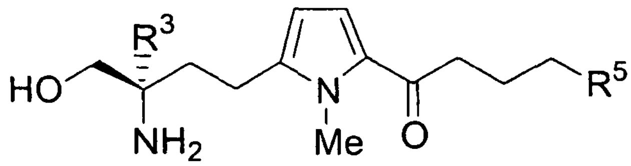

| Compound No. | $R^3$ | $R^5$ |
|---|---|---|
| 141 | Et | 2-Ac-Ph |
| 142 | Et | 3-Ac-Ph |
| 143 | Et | 4-Ac-Ph |
| 144 | Et | 2-CN-Ph |
| 145 | Et | 3-CN-Ph |
| 146 | Et | 4-CN-Ph |

In Table 1 described above, the preferred compounds (I) of the present invention include Exemplification compound numbers: 17-24, 40-47, 54-63, 72, 73, 90-97, 113-120, 127-136, 145 and 146, and the more preferred compounds include
2-amino-2-methyl-4-{1-methyl-5-[4-(2-methylphenyl)butanoyl] pyrrol-2-yl}butan-1-ol,
2-amino-2-methyl-4-{1-methyl-5-[4-(3-methylphenyl)butanoyl] pyrrol-2-yl}butan-1-ol,
2-amino-2-methyl-4-{1-methyl-5-[4-(4-methylphenyl)butanoyl] pyrrol-2-yl}butan-1-ol,
2-amino-2-methyl-4-{1-methyl-5-[4-(2,3-dimethylphenyl)butanoyl] pyrrol-2-yl}butan-1-ol,
2-amino-2-methyl-4-{1-methyl-5-[4-(2,4-dimethylphenyl)butanoyl] pyrrol-2-yl}butan-1-ol,
2-amino-2-methyl-4-{1-methyl-5-[4-(2,5-dimethylphenyl)butanoyl] pyrrol-2-yl}butan-1-ol,
2-amino-2-methyl-4-{1-methyl-5-[4-(3,4-dimethylphenyl)butanoyl] pyrrol-2-yl}butan-1-ol,
2-amino-2-methyl-4-{1-methyl-5-[4-(3,5-dimethylphenyl)butanoyl] pyrrol-2-yl}butan-1-ol,
2-amino-2-methyl-4-{1-methyl-5-[4-(4-ethylphenyl)butanoyl] pyrrol-2-yl}butan-1-ol,
2-amino-2-methyl-4-{1-methyl-5-[4-(4-isopropylphenyl)butanoyl] pyrrol-2-yl}butan-1-ol,
2-amino-2-methyl-4-{1-methyl-5-[4-(4-cyclopropylphenyl)butanoyl] pyrrol-2-yl}butan-1-ol,
2-amino-2-methyl-4-{1-methyl-5-[4-(4-t-butylphenyl)butanoyl] pyrrol-2-yl}butan-1-ol,
2-amino-2-methyl-4-{1-methyl-5-[4-(4-methoxyphenyl)butanoyl] pyrrol-2-yl}butan-1-ol,
2-amino-2-methyl-4-{1-methyl-5-[4-(3,4-dimethoxyphenyl)butanoyl] pyrrol-2-yl}butan-1-ol,
2-amino-2-methyl-4-{1-methyl-5-[4-(3-methyl-4-methoxyphenyl) butanoyl]pyrrol-2-yl}butan-1-ol,
2-amino-2-methyl-4-{1-methyl-5-[4-(3-methoxy-4-methylphenyl) butanoyl]pyrrol-2-yl}butan-1-ol,
2-amino-2-methyl-4-{1-methyl-5-[4-(4-fluorophenyl)butanoyl] pyrrol-2-yl}butan-1-ol,
2-amino-2-methyl-4-{1-methyl-5-[4-(4-chlorophenyl)butanoyl] pyrrol-2-yl}butan-1-ol,
2-amino-2-methyl-4-{1-methyl-5-[4-(4-trifluoromethylphenyl) butanoyl]pyrrol-2-yl}butan-1-ol,
2-amino-2-methyl-4-{1-methyl-5-[4-(4-cyanophenyl)butanoyl] pyrrol-2-yl}butan-1-ol,
2-amino-2-ethyl-4-{1-methyl-5-[4-(2-methylphenyl)butanoyl] pyrrol-2-yl}butan-1-ol,
2-amino-2-ethyl-4-{1-methyl-5-[4-(3-methylphenyl)butanoyl] pyrrol-2-yl}butan-1-ol,
2-amino-2-ethyl-4-{1-methyl-5-[4-(4-methylphenyl)butanoyl] pyrrol-2-yl}butan-1-ol,
2-amino-2-ethyl-4-{1-methyl-5-[4-(2,3-dimethylphenyl)butanoyl] pyrrol-2-yl}butan-1-ol,
2-amino-2-ethyl-4-{1-methyl-5-[4-(2,4-dimethylphenyl)butanoyl] pyrrol-2-yl}butan-1-ol,
2-amino-2-ethyl-4-{1-methyl-5-[4-(2,5-dimethylphenyl)butanoyl] pyrrol-2-yl}butan-1-ol,
2-amino-2-ethyl-4-{1-methyl-5-[4-(3,4-dimethylphenyl)butanoyl] pyrrol-2-yl}butan-1-ol,
2-amino-2-ethyl-4-{1-methyl-5-[4-(3,5-dimethylphenyl)butanoyl] pyrrol-2-yl}butan-1-ol,
2-amino-2-ethyl-4-{1-methyl-5-[4-(4-ethylphenyl)butanoyl] pyrrol-2-yl}butan-1-ol,
2-amino-2-ethyl-4-{1-methyl-5-[4-(4-isopropylphenyl)butanoyl] pyrrol-2-yl}butan-1-ol,
2-amino-2-ethyl-4-{1-methyl-5-[4-(4-cyclopropylphenyl)butanoyl] pyrrol-2-yl}butan-1-ol,
2-amino-2-ethyl-4-{1-methyl-5-[4-(4-t-butylphenyl)butanoyl] pyrrol-2-yl}butan-1-ol,
2-amino-2-ethyl-4-{1-methyl-5-[4-(4-methoxyphenyl)butanoyl] pyrrol-2-yl}butan-1-ol,
2-amino-2-ethyl-4-{1-methyl-5-[4-(3,4-dimethoxyphenyl)butanoyl] pyrrol-2-yl}butan-1-ol,
2-amino-2-ethyl-4-{1-methyl-5-[4-(3-methyl-4-methoxyphenyl) butanoyl]pyrrol-2-yl}butan-1-ol,
2-amino-2-ethyl-4-{1-methyl-5-[4-(4-fluorophenyl)butanoyl] pyrrol-2-yl}butan-1-ol,
2-amino-2-ethyl-4-{1-methyl-5-[4-(4-chlorophenyl)butanoyl] pyrrol-2-yl}butan-1-ol,

2-amino-2-ethyl-4-{1-methyl-5-[4-(4-trifluoromethylphenyl) butanoyl]pyrrol-2-yl}butan-1-ol, and
2-amino-2-ethyl-4-{1-methyl-5-[4-(4-cyanophenyl)butanoyl] pyrrol-2-yl}butan-1-ol, and
the still more preferred compounds include Exemplification compound numbers: 17-24, 54-63, 72 and 73, and the most preferred compounds include
Exemplification compound number 17:
2-amino-2-methyl-4-{1-methyl-5-[4-(2-methylphenyl)butanoyl] pyrrol-2-yl}butan-1-ol,
Exemplification compound number 18:

2-amino-2-methyl-4-{1-methyl-5-[4-(3-methylphenyl)butanoyl] pyrrol-2-yl}butan-1-ol,

Exemplification compound number 19:

2-amino-2-methyl-4-{1-methyl-5-[4-(4-methylphenyl)butanoyl] pyrrol-2-yl}butan-1-ol,

Exemplification compound number 20:

2-amino-2-methyl-4-{1-methyl-5-[4-(2,3-dimethylphenyl)butanoyl] pyrrol-2-yl}butan-1-ol,

Exemplification compound number 21:

2-amino-2-methyl-4-{1-methyl-5-[4-(2,4-dimethylphenyl)butanoyl] pyrrol-2-yl}butan-1-ol,

Exemplification compound number 22:

2-amino-2-methyl-4-{1-methyl-5-[4-(2,5-dimethylphenyl)butanoyl] pyrrol-2-yl}butan-1-ol,

Exemplification compound number 23:

2-amino-2-methyl-4-{1-methyl-5-[4-(3,4-dimethylphenyl)butanoyl] pyrrol-2-yl}butan-1-ol,

Exemplification compound number 24:

2-amino-2-methyl-4-{1-methyl-5-[4-(3,5-dimethylphenyl)butanoyl] pyrrol-2-yl}butan-1-ol,

Exemplification compound number 57:

2-amino-2-methyl-4-{1-methyl-5-[4-(3-methyl-4-methoxyphenyl) butanoyl]pyrrol-2-yl}butan-1-ol,

Exemplification compound number 62:

2-amino-2-methyl-4-{1-methyl-5-[4-(3-methoxy-4-methylphenyl) butanoyl]pyrrol-2-yl}butan-1-ol, and

Exemplification compound number 73:

2-amino-2-methyl-4-{1-methyl-5-[4-(4-cyanophenyl)butanoyl] pyrrol-2-yl}butan-1-ol.

[Advantages of the Invention]

Since medicinal compositions of the present invention exert excellent immunosuppressing effects with low toxicity, the medicinal compositions of the present invention are useful as prophylactic or therapeutic agents for immune-related diseases such as reject reactions caused by organ or skin transplantation, rheumatic arthritis, and other autoimmune diseases in mammals (particularly in humans).

[Best mode for carrying out the Invention]

HMG-CoA reductase inhibitors used as an active ingredient in the medicinal composition of the present invention can easily be prepared according to the methods from Patent literature 8 to Patent literature 14 described hereinbefore (Japanese Patent Publication (Kokai) Number Sho 57-2240 (USP 4346227), Japanese Patent Publication (Kokai)

Number Sho 57-163374 (USP 4231938), Japanese Patent Publication (Kokai) Number Sho 56-122375 (USP 4444784), Japanese Patent Publication (Kohyo) Number Sho 60-500015 (USP 4739073), Japanese Patent Publication (Kokai) Number Hei3-58967 (USP5273995), Japanese Patent Publication (Kokai) Number Hei 5-178841 (USP 5260440), Japanese Patent Publication (Kokai) Number Hei 1-279866 (USP 5854259 and USP 5856336)), or the like.

In addition, the amino alcohol derivatives, which are one of the active ingredients of the medicinal composition of the present invention, can easily be prepared, for example, according to the methods from Patent literature 1 to Patent literature 7 described hereinbefore (WO 94/08943 (EP627406), WO96/06068, WO98/45249, WO03/029184, WO03/029205, Japanese Patent Publication (Kokai) Number 2002-167382, Japanese Patent Publication (Kokai) Number 2003-267950) and the like.

The medicinal compositions of the present invention which contain both an HMG-CoA reductase inhibitor and an amino alcohol derivative augment the pharmacological immunosuppressive activity and lower its side effect and toxicity. Thus the medicinal composition of the present invention is useful as a prophylactic or therapeutic agent for autoimmune diseases such as systemic lupus erythematosus, rheumatoid arthritis, polymyositis, dermatomyositis, scleroderma, Behcet's syndrome, Crohn's disease, ulcerative colitis, autoimmune hepatitis, aplastic anemia, idiopathic thrombocytopenic purpura, autoimmune hemolytic anemia, multiple sclerosis, autoimmune bullosis, psoriasis vulgaris, vasculitis syndrome, Wegener's granuloma, uveitis, idiopathic interstitial pneumonia, Goodpasture's syndrome, sarcoidosis, allergic granulomatous angitis, bronchial asthma, myocarditis, cardiomyopathy, aortitis syndrome, postmyocardial infarction syndrome, primary pulmonary hypertension, minimal change nephrotic syndrome, membranous nephropathy, membranoproliferative glomerulonephritis, focal glomerular sclerosis, crescentic glomerulonephritis, myasthenia gravis, inflammatory neuropathy, atopic dermatitis, chronic actinic dermatitis, acute polyarthritis, Sydenham's chorea, systemic sclerosis, adult-onset type diabetes mellitus, insulin dependent diabetes mellitus, juvenile diabetes mellitus, atherosclerosis, glomerular nephritis, tubulointerstitial nephritis, primary biliary cirrhosis, primary sclerosing cholangitis, fulminant hepatitis, viral hepatitis, GVHD, reject reactions caused by transplantation of various organs, contact dermatitis, and sepsis, or other immunology-related diseases.

In the case that the medicinal composition of the present invention is used as a prophylactic or therapeutic agent for diseases described above, the medicinal composition of the present invention can be administered in a suitable dosage form by mixing with a suitable pharmacologically acceptable excipient and/or diluent, for example, as tablets, capsules, granules, powders, or syrups for oral administration, or injections or suppositories for parenteral administration.

The medicinal compositions are prepared, according to well known techniques, using additives such as excipients (for example, organic excipients including sugar derivatives such as lactose, sucrose, glucose, mannitol, and sorbitol; starch derivatives such as corn starch, potato starch, α-starch, and dextrin; cellulose derivatives such as crystalline cellulose; gum arabic; dextran; pullulan; and inorganic excipients including silicate derivatives such as light anhydrous silicic acid, synthetic aluminum silicate, calcium silicate, and magnesium aluminometasilicate; phosphates such as calcium hydrogenphosphate; carbonates such as calcium carbonate; and sulfates such as calcium sulfate can be listed), lubricants (for example, stearic acid and metal salts of stearic acid such as calcium stearate and magnesium stearate; talc; colloidal silica; waxes such as veegum and spermaceti; boric acid; adipic acid; sulfates such as sodium sulfate; glycol; fumaric acid; sodium benzoate; DL-leucine; sodium fatty acid; lauryl sulfates such as sodium lauryl sulfate and magnesium lauryl sulfate; silicates such as silicic anhydride and silicic hydrate; and the starch derivatives described above can be listed), binders (for example, hydroxypropylcellulose, hydroxypropylmethylcellulose, polyvinylpyrrolidone, Macrogol, and similar excipients to those described above can be listed), disintegrants (for example, cellulose derivatives such as low-substituted hydroxypropylcellulose, carboxymethylcellulose, calcium carboxymethylcellulose, and internally crosslinked sodium carboxymethylcellulose; and chemically modified starch/cellulose derivatives such as carboxymethylstarch, sodium carboxymethylstarch, and crosslinked polyvinylpyrrolidone can be listed), stabilizers (for example, paraoxybenzoates such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid can be listed), flavourings (for example, conventionally employed sweeteners, acidifiers, and flavourings can be listed), diluents, and the like.

In the administration of the medicinal composition of the present invention, at least one HMG-CoA reductase inhibitor selected from the specified group described hereinbefore and at least one compound selected from the group consisting of amino alcohol derivatives having the general formula (I) described hereinbefore, pharmacologically acceptable salts thereof and pharmacologically acceptable esters thereof can be administered simultaneously, sequentially or separately on different occasions. In clinical practice, however, it is convenient to administer these drugs at the same time, and accordingly, said HMG-CoA reductase inhibitor and at least one compound selected from the group consisting of amino alcohol derivatives having the general formula (I) described hereinbefore, pharmacologically acceptable salts thereof and pharmacologically acceptable esters thereof can be administered in the form of a combination drug.

When it is undesirable to mix the pharmaceutical preparations of both drugs together physically, it is possible to administer these drugs simultaneously as a kit comprising the pharmaceutical preparations of each drug, and it is also possible to administer each of the pharmaceutical preparations of these drugs one after the other at a suitable

interval, since these drugs exert a prominent effect even if these drugs are not administered simultaneously.

In the present invention, a simultaneous administration is not particularly restricted provided that these drugs have a pharmaceutical dose form that can be administered at almost the same time, but it is preferable to administer them as a single medicinal composition.

In the present invention, sequential or separate administration is not particularly restricted provided that these drugs have a pharmaceutical dose form that can be administered separately at different times, and means, for example, that at least one compound selected from the group consisting of amino alcohol derivatives having the general formula (I) described hereinbefore, pharmacologically acceptable salts thereof and pharmacologically acceptable esters thereof is administered first and then at least one HMG-CoA reductase inhibitor selected from the specified group described hereinbefore is administered after a defined time, or that said HMG-CoA reductase inhibitor is administered first and then at least one compound selected from the group consisting of amino alcohol derivatives having the general formula (I) described hereinbefore, pharmacologically acceptable salts thereof and pharmacologically acceptable esters thereof is administered after a defined time. In the present invention, said HMG-CoA reductase inhibitor and at least one compound selected from the group consisting of amino alcohol derivatives having the general formula (I) described hereinbefore, pharmacologically acceptable salts thereof and pharmacologically acceptable esters thereof can be administered separately, and the maximum time interval, between administration of these drugs, acceptable for achieving said prominent effect by these drugs can be confirmed in clinical practice or by animal experiments.

Each dose of at least one HMG-CoA reductase inhibitor selected from the specified group described hereinbefore and at least one compound selected from the group consisting of amino alcohol derivatives having the general formula (I) described hereinbefore, pharmacologically acceptable salts thereof and pharmacologically acceptable esters thereof, both of which are active ingredients of the present invention, and the dose ratio between these drugs, may vary depending on a variety of factors such as the activity of each drug, and the symptoms, age and body weight of the patient.

The dose may vary depending on a variety of factors such as the symptoms and age of the patient. For example, in the case of oral administration, the dose is between 0.05 mg and 200 mg per one time for a human adult (body weight: about 60 kg), and is preferably between 5 mg and 40 mg. In the case of intravenous administration, the dose is between 0.01 mg and 100 mg per one time for a human adult (body weight: about 60 kg), and is preferably between 1 mg and 10 mg. The dosing frequency is from one to six times per day for a human adult (body weight: about 60 kg), and the defined daily dosage may be administered throughout the day at the same time or at certain intervals depending on the symptoms of the patient.

The dose ratio between at least one agent selected from HMG-CoA reductase inhibitors defined hereinbefore and at least one compound selected from the group consisting of the amino alcohol derivatives having the general formula (I) described hereinbefore, pharmacologically acceptable salts thereof and pharmacologically acceptable esters thereof, both of which are active ingredients of medicinal compositions of the present invention, may also vary considerably, but the dose ratio by weight between said HMG-CoA reductase inhibitor and at least one compound selected from the group of the amino alcohol derivatives having the general formula (I) described hereinbefore, pharmacologically acceptable salts thereof and pharmacologically acceptable esters thereof may be within a range between 1:500 and 500:1.

[Examples]

(Test Example 1)

Determination of Inhibitory Activities of the Compound against Host versus Graft Reaction in the Rat

(1) Two strains of rats [Lewis rats (male, 6 weeks of age, Charles River Japan Inc.) and WKAH/Hkm rats (male, 7 weeks of age, Japan SLC Inc.) are used. Five rats (host) per group are used.

(2) Induction of HvGR

Spleen cells are isolated from the spleens of WKAH/Hkm or Lewis rats and floated in RPMI1640 medium (Life Technologies Inc.) at a concentration of $1 \times 10^8$ cells/ml. 0.1 ml of the medium containing the free-floating spleen cells of WKAH/Hkm rats is then intracutaneously injected into the bilateral foot-pads of the hindlimbs of both sides of Lewis rats (HvGR induction group) or that of Lewis rats is then intracutaneously injected into the bilateral foot-pads of the hindlimbs of both sides of Lewis rats (the same strain group).

(3) Administration of the Compound

The test compound is suspended in 0.5% tragacantha solution at concentrations of 0.8 mg/5 ml, 0.08 mg/5

ml, and 0.008 mg/5 ml.

In groups of rats administered with a statin and an immunosuppressant, a suspended solution of a statin and an immunosuppressant is orally administered at a volume of 5 ml/kg, once daily, for 4 successive days starting on the day of the spleen cell injection.

In addition, a statin suspended solution and tragacantha solution (0.5%) is orally administered to rats in a "statin administration group", while an immunosuppressant and tragacantha solution (0.5%) is orally administered to rats in an "immunosuppressant administration group". The administration volumes in the rats of both of these 2 groups are 5 ml/kg, once daily, for 4 successive days starting on the day of the spleen cell injection.

Furthermore, tragacantha solution (0.5%) is orally administered to rats in the "same strain group" (Lewis rats injected with spleen cells of Lewis rats, but not treated with the compound) and the control group (Lewis rats injected with spleen cells of WKAH/Hkm rats and not treated with the compound).

(4) Determination of Inhibitory Activity against HvGR

The average weight of the popliteal lymph nodes of the same strain rats is subtracted from the individual weights of the popliteal lymph nodes of individual rats ("Weight of the popliteal lymph nodes after HvGR-induction"). The inhibitory activities of compounds are calculated from the "Weight of the popliteal lymph nodes after HvGR-induction" of individual rats in the drug-treated group versus the average "Weight of the popliteal lymph nodes after HvGR-induction" in the control group (Equation 1):

$$\text{Inhibitory Rate(\%)} = \frac{\text{(Average “Weight of the popliteal lymph nodes after HvGR-induction” in control group) - (Average “Weight of the popliteal lymph nodes after HvGR-induction” in drug-treated group)}}{\text{(Average “Weight of the popliteal lymph nodes after HvGR-induction” in control group)}} \times 100$$

(Test Example 2)

Arthritis Development Preventative Activity

The inhibitory activity of the medicinal composition of the present invention against arthritis is assessed by using the rate of inhibition of swelling of the right hind paw as an indicator in an adjuvant-induced arthritis model which exhibits symptoms similar to human arthritis. Female Lewis rats aged 8 weeks are used in this study.

(1) Preparation of adjuvant

Heat-killed dried Mycobacterium butyricum is ground on an agate mortar, and is then suspended in dry-sterilized liquid paraffin to make a 2 mg/ml suspension. The resulting suspended solution is then sonicated and used as adjuvant.

(2) Preparation of Compounds

Compounds are dissolved or suspended in 0.5% tragacantha solution.

(3) Induction of Adjuvant-Induced Arthritis

Arthritis is induced by intradermal injection of the adjuvant prepared in (1) described above into the heel of the right hind paw of rats in the drug-treated group and in the control group. Five rats per group are used. Non-treated rats are separately used as a normal control group.

(4) Administration of the Compound

The test compounds prepared in (2) described above are orally administered to the rats of the compound-treated group at a volume of 5 ml/kg, once a day from the injection day of the adjuvant (day 0) for 18 successive days. 0.5% tragacantha solution alone is similarly administered to rats in the control groups.

(5) Calculation of Inhibition Rate of Swollen Foot Volume by the Test Compound

On the 11th and 18th day after the drug administration is started, the right foot volume of each rat is measured by an apparatus for determination of the volume. The average swelling volume of each group is calculated.

The percent inhibition of the swelling of the injected foot of the treated animals as compared to that of the control animals is calculated according to the following equation:

Inhibition rate of swollen foot volume (%) = {1-[(swollen foot volume of compound-treated animals) - (swollen foot volume of normal control animals)] / [(swollen foot volume of control animals)-( swollen foot volume of normal control animals)]} × 100

(Test Example 3)

Assessment of Inhibitory Activity against Peripheral Lymphocytes of the Rat

Lewis rats (male, 5 weeks of age, Charles River Japan Inc.) are used. Five rats per group are used.

(1) Administration of the Compound

The test compound is suspended in 1% tragacantha solution (vehicle). The suspended solution of the test compound is orally administered to rats at a volume of 5 ml/kg.

In control rats, vehicle is orally administered, instead of the suspended solution of the compound.

(2) Counting of Peripheral Lymphocytes

3 hours after administration of either the vehicle or the suspended solution of the test compound, blood is collected from the postcaval vein of the rats under ether anesthesia. Then the collected blood is transferred into a tube containing EDTA. The absolute number of lymphocytes in the blood is counted using a full blood count analyser. The inhibitory activity (%) of the test compound is determined by calculation of the relative number of peripheral lymphocytes with the number of lymphocytes from normal rats being defined as 100%.

(Formulation Examples)

Tablets

| | |
|---|---|
| A statin | 50.0 mg |
| An immunosuppressant | 10.0 mg |
| Lactose | 113.0 mg |
| Corn starch | 25.0 mg |
| Magnesium stearate | 2.0 mg |
| | 200.0 mg |

Tablets (200 mg in a tablet) are prepared by mixing powders of the above prescription in a blender, and a

tableting the mixture using a tableting machine.

## Claims

1. A medicinal composition comprising at least one agent selected from the group consisting of HMG-CoA reductase inhibitors and at least one compound selected from the group consisting of amino alcohol derivatives having the general formula (I):

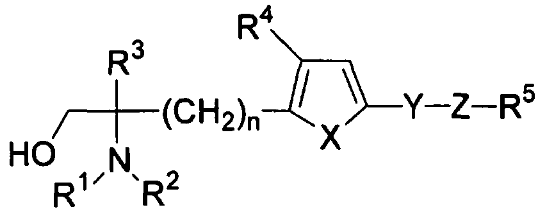

(I)

[wherein,
$R^1$ and $R^2$ are the same or different and each represents a hydrogen atom or a lower alkyl group,
$R^3$ represents a lower alkyl group, or a hydroxymethyl group,
$R^4$ represents a hydrogen atom, a lower alkyl group, a lower alkoxy group, or a halogen atom,
$R^5$ represents a hydrogen atom, a halogen atom, a cyclohexyl group,
a phenyl group, or a phenyl group substituted with from 1 to 3 substituents selected from Substituent group a,
X represents a methylated nitrogen atom,
Y represents a single bond, an oxygen atom, a sulfur atom, or a carbonyl group,
Z represents a single bond, a $C_1$-$C_8$ alkylene group, or a $C_1$-$C_8$ alkylene group substituted with from 2 to 8 fluorine atoms,
n represents an integer of 2 or 3,
Substituent group a represents the group consisting of a halogen atom,
a cyano group, a lower alkyl group, a lower cycloalkyl group, a lower alkoxy group, a trifluoromethyl group, a phenyl group, and a benzyloxy group,
the "lower alkyl group" is a methyl, ethyl, propyl, isopropyl, butyl, isobutyl, s-butyl, t-butyl, pentyl or hexyl group,
the "lower alkoxy group" is a methoxy, ethoxy, propoxy, isopropoxy, butoxy, isobutoxy, s-butoxy, t-butoxy, pentoxy or hexyloxy group, and
the "lower cycloalkyl group" is a cyclopropyl, cyclobutyl, cyclopentyl or cyclohexyl group], pharmacologically acceptable salts thereof, and pharmacologically acceptable esters thereof.

2. A medicinal composition according to Claim 1, in which an amino alcohol derivative having the general formula (I) is a compound having the general formula (Ia) shown below,

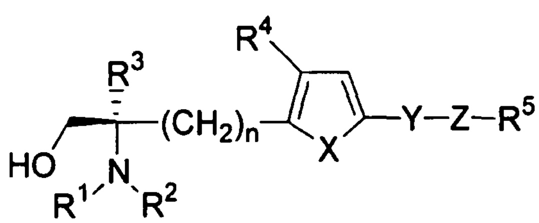

(Ia)

3. A medicinal composition according to Claim 1 or Claim 2, in which the HMG-CoA reductase inhibitor is at least one agent selected from the group consisting of pravastatin, lovastatin, simvastatin, fluvastatin, atorvastatin, rosuvastatin, and pitavastatin.

4. A medicinal composition according to Claim 1 or Claim 2, in which the HMG-CoA reductase inhibitor is pravastatin.

5. A medicinal composition according to Claim 1 or Claim 2, in which the HMG-CoA reductase inhibitor is atorvastatin.

6. A medicinal composition according to any one claim selected from Claim 1 to Claim 5, in which $R^1$ and $R^2$ are a hydrogen atom.

7. A medicinal composition according to any one claim selected from Claim 1 to Claim 6, in which $R^3$ is a methyl group, an ethyl group, or a hydroxymethyl group.

8. A medicinal composition according to any one claim selected from Claim 1 to Claim 6, in which $R^3$ is a hydroxymethyl group.

9. A medicinal composition according to any one claim selected from Claim 1 to Claim 6, in which $R^3$ is a methyl group.

10. A medicinal composition according to any one claim selected from Claim 1 to Claim 9, in which $R^4$ is a hydrogen atom.

11. A medicinal composition according to any one claim selected from Claim 1 to Claim 10, in which $R^5$ is a phenyl group substituted with from 1 to 3 substituents selected from the group consisting of a fluorine atom, a cyano group, a methyl group, a methoxy group, an ethoxy group, and a phenyl group.

12. A medicinal composition according to any one claim selected from Claim 1 to Claim 10, in which $R^5$ is a phenyl group substituted with from 1 to 3 substituents selected from the group consisting of a methyl group and a methoxy group.

13. A medicinal composition according to any one claim selected from Claim 1 to Claim 10, in which $R^5$ is a phenyl group.

14. A medicinal composition according to any one claim selected from Claim 1 to Claim 13, in which Y represents a carbonyl group.

15. A medicinal composition according to any one claim selected from Claim 1 to Claim 14, in which Z represents a single bond, or a $C_1$-$C_8$ alkylene group.

16. A medicinal composition according to any one claim selected from Claim 1 to Claim 15, in which n represents an integer of 2.

17. A medicinal composition according to Claim 1, in which an amino alcohol derivative having the general formula (I) is the following compound:

2-amino-2-methyl-4-{1-methyl-5-[4-(2-methylphenyl)butanoyl] pyrrol-2-yl}butan-1-ol,
2-amino-2-methyl-4-{1-methyl-5-[4-(3-methylphenyl)butanoyl] pyrrol-2-yl}butan-1-ol,
2-amino-2-methyl-4-{1-methyl-5-[4-(4-methylphenyl)butanoyl] pyrrol-2-yl}butan-1-ol,
2-amino-2-methyl-4-{1-methyl-5-[4-(2,3-dimethylphenyl)butanoyl] pyrrol-2-yl}butan-1-ol,
2-amino-2-methyl-4-{1-methyl-5-[4-(2,4-dimethylphenyl)butanoyl] pyrrol-2-yl}butan-1-ol,
2-amino-2-methyl-4-{1-methyl-5-[4-(2,5-dimethylphenyl)butanoyl] pyrrol-2-yl}butan-1-ol,
2-amino-2-methyl-4-{1-methyl-5-[4-(3,4-dimethylphenyl)butanoyl] pyrrol-2-yl}butan-1-ol,
2-amino-2-methyl-4-{1-methyl-5-[4-(3,5-dimethylphenyl)butanoyl] pyrrol-2-yl}butan-1-ol,
2-amino-2-methyl-4-{1-methyl-5-[4-(3-methyl-4-methoxyphenyl) butanoyl]pyrrol-2-yl}butan-1-ol,
2-amino-2-methyl-4-{1-methyl-5-[4-(3-methoxy-4-methylphenyl) butanoyl]pyrrol-2-yl}butan-1-ol,
2-amino-2-methyl-4-{1-methyl-5-[4-(4-cyanophenyl)butanoyl] pyrrol-2-yl}butan-1-ol,
2-amino-2-methyl-4-{1-methyl-5-[5-(2-methylphenyl)pentanoyl] pyrrol-2-yl}butan-1-ol,
2-amino-2-methyl-4-{1-methyl-5-[5-(3-methylphenyl)pentanoyl] pyrrol-2-yl}butan-1-ol,
2-amino-2-methyl-4-{1-methyl-5-[5-(4-methylphenyl)pentanoyl] pyrrol-2-yl}butan-1-ol,
2-amino-2-methyl-4-{1-methyl-5-[5-(2,3-dimethylphenyl)pentanoyl] pyrrol-2-yl}butan-1-ol,
2-amino-2-methyl-4-{1-methyl-5-[5-(2,4-dimethylphenyl)pentanoyl] pyrrol-2-yl}butan-1-ol,
2-amino-2-methyl-4-{1-methyl-5-[5-(2,5-dimethylphenyl)pentanoyl] pyrrol-2-yl}butan-1-ol,
2-amino-2-methyl-4-{1-methyl-5-[5-(3,4-dimethylphenyl)pentanoyl] pyrrol-2-yl}butan-1-ol,
2-amino-2-methyl-4-{1-methyl-5-[5-(3,5-dimethylphenyl)pentanoyl] pyrrol-2-yl}butan-1-ol,
2-amino-2-methyl-4-{1-methyl-5-[5-(3-methyl-4-methoxyphenyl) pentanoyl]pyrrol-2-yl}butan-1-ol,
2-amino-2-methyl-4-{1-methyl-5-[5-(3-methoxy-4-methylphenyl) pentanoyl]pyrrol-2-yl}butan-1-ol, or
2-amino-2-methyl-4-{1-methyl-5-[5-(4-cyanophenyl)pentanoyl] pyrrol-2-yl}butan-1-ol.

**18.** A medicinal composition according to any one claim selected from Claim 1to Claim 17, in the form of a combination drug for use in the treatment or prophylaxis of an autoimmune disease.

**19.** A medicinal composition according to any one claim selected from Claim 1 to Claim 17, in the form of a kit for use in the treatment or prophylaxis of an autoimmune disease, wherein said kit comprises a pharmaceutical preparation of said at least one HMG-CoA reductase inhibitor agent and a pharmaceutical preparation of said at least one amino alcohol derivative compound.

**20.** Use of a medicinal composition according to any one claim selected from Claim 1 to Claim 17 for the manufacture of a medicament for prophylaxis or treatment of an autoimmune disease.

**21.** Use of at least one agent selected from the group of HMG-CoA reductase inhibitors and at least one compound selected from the group consisting of amino alcohol derivatives having the general formula (I), pharmacologically acceptable salts thereof and pharmacologically acceptable esters thereof as defined in any one claim selected from Claim 1 to Claim 17 for the manufacture of a medicament for administering said at least one agent and said at least one compound simultaneously, sequentially or separately on different occasions for prophylaxis or treatment of an autoimmune disease.

**22.** A method of preparing a medicinal composition according to any one claim selected from Claim 1 to Claim 17, which comprises mixing at least one agent selected from the group of HMG-CoA reductase inhibitors and at least one compound selected from the group consisting of amino alcohol derivatives having the general formula (I), pharmaceutically acceptable salts thereof and pharmacologically acceptable esters thereof.

**Patentansprüche**

**1.** Medizinische Zusammensetzung, umfassend mindestens ein Agens, das aus der Gruppe bestehend aus HMG-CoA Reduktase-Inhibitoren ausgewählt ist, und mindestens eine Verbindung, die aus der Gruppe bestehend aus Aminoalkoholderivaten mit der folgenden allgemeinen Formel (I) ausgewählt ist:

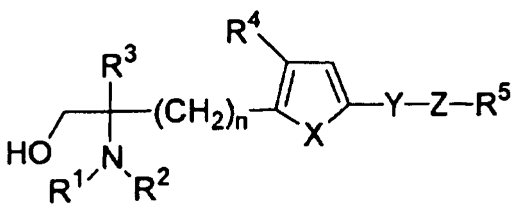

(I)

[wobei
$R^1$ und $R^2$ gleich oder verschieden sind und jeweils ein Wasserstoffatom oder eine niedere Alkylgruppe darstellen,
$R^3$ eine niedere Alkylgruppe oder eine Hydroxymethylgruppe darstellt,
$R^4$ ein Wasserstoffatom, eine niedere Alkylgruppe, eine niedere Alkyoxygruppe oder ein Halogenatom darstellt,
$R^5$ ein Wasserstoffatom, ein Halogenatom, eine Cyclohexylgruppe, eine Phenylgruppe oder eine Phenylgruppe, die mit 1 bis 3 Substituenten substituiert ist, die aus der Substituentengruppe a ausgewählt sind, darstellt,
X ein methyliertes Stickstoffatom darstellt,
Y eine Einfachbindung, ein Sauerstoffatom, ein Schwefelatom oder eine Carbonylgruppe darstellt,
Z eine Einfachbindung, eine $C_1$-$C_8$-Alkylengruppe oder eine $C_1$-$C_8$-Alkylengruppe, die mit 2 bis 8 Fluoratomen substituiert ist, darstellt,
n eine ganze Zahl von 2 oder 3 darstellt,
die Substituentengruppe a die Gruppe darstellt, die aus einem Halogenatom, einer Cyanogruppe, einer niederen Alkylgruppe, einer niederen Cycloalkylgruppe, einer niederen Alkoxygruppe, einer Trifluormethylgruppe, einer Phenylgruppe und einer Benzyloxygruppe besteht,
die "niedere Alkylgruppe" eine Methyl-, Ethyl-, Propyl-, Isopropyl-, Butyl-, Isobutyl-, s-Butyl-, t-Butyl-, Pentyl- oder Hexylgruppe ist,
die "niedere Alkoxygruppe" eine Methoxy-, Ethoxy-, Propoxy-, Isopropoxy-, Butoxy-, Isobutoxy-, s-Butoxy-, t-Butoxy-, Pentoxy- oder Hexyloxygruppe ist, und
die "niedere Cycloalkylgruppe" eine Cyclopropyl-, Cyclobutyl-, Cyclopentyl-oder Cyclohexylgruppe ist], pharmazeutisch akzeptable Salze davon und pharmazeutisch akzeptable Ester davon.

2. Medizinische Zusammensetzung nach Anspruch 1, wobei ein Aminoalkoholderivat mit der allgemeinen Formel (I) eine Verbindung mit der allgemeinen Formel (Ia) ist, die im Folgenden dargestellt ist:

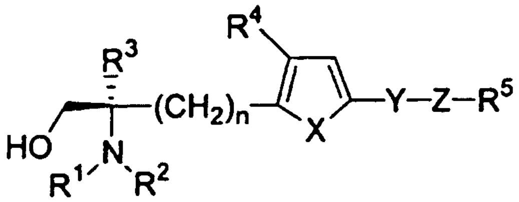

(Ia)

3. Medizinische Zusammensetzung nach Anspruch 1 oder 2, wobei der HMG-CoA Reduktase-Inhibitor mindestens ein Agens ist, das aus der Gruppe bestehend aus Pravastatin, Lovastatin, Simvastatin, Fluvastatin, Atorvastatin, Rosuvastatin und Pitavastatin ausgewählt ist.

4. Medizinische Zusammensetzung nach Anspruch 1 oder 2, wobei der HMG-CoA Reduktase-Inhibitor Pravastatin ist.

5. Medizinische Zusammensetzung nach Anspruch 1 oder 2, wobei der HMG-CoA Reduktase-Inhibitor Atorvastatin ist.

6. Medizinische Zusammensetzung nach einem der Ansprüche 1 bis 5, wobei $R^1$ und $R^2$ ein Wasserstoffatom sind.

7. Medizinische Zusammensetzung nach einem der Ansprüche 1 bis 6, wobei $R^3$ eine Methylgruppe, eine Ethylgruppe oder eine Hydroxymethylgruppe ist.

8. Medizinische Zusammensetzung nach einem der Ansprüche 1 bis 6, wobei $R^3$ eine Hydroxymethylgruppe ist.

9. Medizinische Zusammensetzung nach einem der Ansprüche 1 bis 6, wobei $R^3$ eine Methylgruppe ist.

10. Medizinische Zusammensetzung nach einem der Ansprüche 1 bis 9, wobei $R^4$ ein Wasserstoffatom ist.

11. Medizinische Zusammensetzung nach einem der Ansprüche 1 bis 10, wobei $R^5$ eine Phenylgruppe ist, die mit 1 bis 3 Substituenten substituiert ist, die aus der Gruppe bestehend aus einem Fluoratom, einer Cyanogruppe, einer Methylgruppe, einer Methoxygruppe, einer Ethoxygruppe und einer Phenylgruppe ausgewählt sind.

12. Medizinische Zusammensetzung nach einem der Ansprüche 1 bis 10, wobei $R^5$ eine Phenylgruppe ist, die mit 1 bis 3 Substituenten substituiert ist, die aus der Gruppe bestehend aus einer Methylgruppe und einer Methoxygruppe ausgewählt sind.

13. Medizinische Zusammensetzung nach einem der Ansprüche 1 bis 10, wobei $R^5$ eine Phenylgruppe ist.

14. Medizinische Zusammensetzung nach einem der Ansprüche 1 bis 13, wobei Y eine Carbonylgruppe darstellt.

15. Medizinische Zusammensetzung nach einem der Ansprüche 1 bis 14, wobei Z eine Einfachbindung oder eine $C_1$-$C_8$-Alkylengruppe darstellt.

16. Medizinische Zusammensetzung nach einem der Ansprüche 1 bis 15, wobei n eine ganze Zahl von 2 darstellt.

17. Medizinische Zusammensetzung nach Anspruch 1, wobei ein Aminoalkoholderivat mit der allgemeinen Formel (I) die folgende Verbindung ist:

2-Amino-2-methyl-4-{1-methyl-5-[4-(2-methylphenyl)butanoyl]pyrrol-2-yl}butan-1-ol,
2-Amino-2-methyl-4-{1-methyl-5-[4-(3-methylphenyl)butanoyl]pyrrol-2-yl} butan-1-ol,
2-Amino-2-methyl-4-{1-methyl-5-[4-(4-methylphenyl)butanoyl]pyrrol-2-yl}butan-1-ol,
2-Amino-2-methyl-4- {1-methyl-5-[4-(2,3-dimethylphenyl)butanoyl]pyrrol-2-yl}butan-1-ol,
2-Amino-2-methyl-4- {1-methyl-5-[4-(2,4-dimethylphenyl)butanoyl]pyrrol-2-yl}butan-1-ol,

2-Amino-2-methyl-4-{1-methyl-5-[4-(2,5-dimethylphenyl)butanoyl]pyrrol-2-yl}butan-1-ol,
2-Amino-2-methyl-4-{1-methyl-5-[4-(3,4-dimethylphenyl)butanoyl]pyrrol-2-yl}butan-1-ol,
2-Amino-2-methyl-4- {1-methyl-5 - [4-(3,5-dimethylphenyl)butanoyl] pyrrol -2-yl}butan-1-ol,
2-Amino-2-methyl-4-{ 1-methyl-5-[4-(3-methyl-4-methoxyphenyl)butanoyl]pyrrol-2-yl}butan-1-ol,
2-Amino-2-methyl-4-{(1-methyl-5-(4-(3-methoxy-4-methylphenyl)butanoyl]pyrrol-2-yl} butan-1-ol,
2-Amino-2-methyl-4-{1-methyl-5-[4-(4-cyanophenyl)butanoyl]pyrrol-2-yl}butan-1-ol,
2-Amino-2-methyl-4-{l-methyl-5-[5-(2-methylphenyl)pentanoyl]pyrrol-2-yl}butan-1-ol,
2-Amino-2-methyl-4-{1-methyl-5-[5-(3-methylphenyl)pentanoyl]pyrrol-2-yl} butan-1-ol,
2-Amino-2-methyl-4-{1-methyl-5-[5-(4-methylphenyl)pentanoyl]pyrrol-2-yl}butan-1-ol,
2-Amino-2-methyl-4-{1-methyl-5-[5-(2,3-dimethylphenyl)pentanoyl]pyrrol-2-yl}butan-1-ol,
2-Amino-2-methyl-4-{1-methyl-5-[5-(2,4-dimethylphenyl)pentanoyl]pyrrol-2-yl}butan-1-ol,
2-Amino-2-methyl-4-{1-methyl-5-[5-(2,5-dimethylphenyl)pentanoyl]pyrrol-2-yl}butan-1-ol,
2-Amino-2-methyl-4-{1-methyl-5-[5-(3,4-dimethylphenyl)pentanoyl]pyrrol-2-yl}butan-1-ol,
2-Amino-2-methyl-4-{1-methyl-5-[5-(3,5-dimethylphenyl)pentanoyl]pyrrol-2-yl}butan-1-ol,
2-Amino-2-methyl-4-{1-methyl-5-[5-(3 -methyl-4-methoxyphenyl)pentanoyl]pyrrol-2-yl}butan-1-ol,
2-Amino-2-methyl-4-{1-methyl-5-(5-(3-methoxy-4-methylphenyl)pentanoyl]pyrrol-2-yl}butan-1-ol oder
2-Amino-2-methyl-4-{1-methyl-5-[5-(4-cyanophenyl)pentanoyl]pyrrol-2-yl}butan-1-ol.

**18.** Medizinische Zusammensetzung nach einem der Ansprüche 1 bis 17 in Form eines Kombinationsarzneimittels zur Verwendung bei der Behandlung oder Verhütung einer Autoimmunkrankheit.

**19.** Medizinische Zusammensetzung nach einem der Ansprüche 1 bis 17 in Form eines Kits zur Verwendung bei der Behandlung oder Verhütung einer Autoimmunkrankheit, wobei der Kit ein pharmazeutisches Präparat des mindestens einen HMG-CoA Reduktase-Inhibitor-Agens und ein pharmazeutisches Präparat der mindestens einen Aminoalkoholderivat-Verbindung umfasst.

**20.** Medizinische Zusammensetzung nach einem der Ansprüche 1 bis 17 zur Herstellung eines Medikaments zur Verhütung oder Behandlung einer Autoimmunkrankheit.

**21.** Verwendung von mindestens einem Agens, das aus der Gruppe von HMG-CoA Reduktase-Inhibitoren ausgewählt ist, und mindestens einer Verbindung, die aus der Gruppe bestehend aus Aminoalkoholderivaten mit der allgemeinen Formel (I) ausgewählt ist, pharmazeutisch akzeptablen Salzen davon und pharmazeutisch akzeptablen Estern davon nach einem der Ansprüche 1 bis 17 zur Herstellung eines Medikaments für die Verabreichung des mindestens einen Agens und der mindestens einen Verbindung gleichzeitig, aufeinander folgend oder separat bei verschiedenen Gelegenheiten zur Verhütung oder Behandlung einer Autoimmunkrankheit.

**22.** Verfahren zur Herstellung einer medizinischen Zusammensetzung nach einem der Ansprüche 1 bis 17, welches ein Mischen von mindestens einem Agens, das aus der Gruppe von HMG-CoA Reduktase-Inhibitoren ausgewählt ist, und mindestens einer Verbindung, die aus der Gruppe bestehend aus Aminoalkoholderivaten mit der allgemeinen Formel (I) ausgewählt ist, pharmazeutisch akzeptablen Salzen davon und pharmazeutisch akzeptablen Estern davon umfasst.

## Revendications

**1.** Composition médicinale comprenant au moins un agent sélectionné parmi le groupe consistant en inhibiteurs de la HMG-CoA réductase et au moins un composé sélectionné parmi le groupe consistant en dérivés d'alcool aminé ayant la formule générale (I):

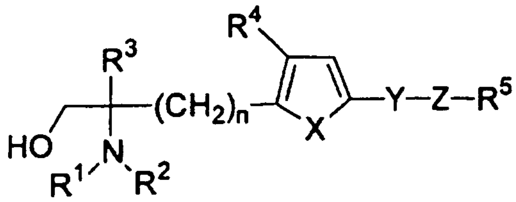

(I)

[où,

$R^1$ et $R^2$ sont les mêmes ou différents et représentent chacun un atome d'hydrogène ou un groupe alkyle inférieur,
$R^3$ représente un groupe alkyle inférieur ou un groupe hydroxyméthyle,
$R^4$ représente un atome d'hydrogène, un groupe alkyle inférieur, un groupe alcoxy inférieur ou un atome d'halogène,
$R^5$ représente un atome d'hydrogène, un atome d'halogène, un groupe cyclohexyle, un groupe phényle ou un groupe phényle substitué par 1 à 3 substituants sélectionnés parmi le groupe substituant a,
X représente un atome d'azote méthylé,
Y représente une liaison simple, un atome d'oxygène, un atome de soufre ou un groupe carbonyle,
Z représente une liaison simple, un groupe alkylène $C_1$-$C_8$ ou un groupe alkylène $C_1$-$C_8$ substitué par 2 à 8 atomes de fluor,
n représente un entier de 2 ou 3,
le groupe substituant a représente le groupe consistant en un atome d'halogène, un groupe cyano, un groupe alkyle inférieur, un groupe cycloalkyle inférieur, un groupe alcoxy inférieur, un groupe trifluorométhyle, un groupe phényle et un groupe benzyloxy,
le "groupe alkyle inférieur" est un groupe méthyle, éthyle, propyle, isopropyle, butyle, isobutyle, s-butyle, t-butyle, pentyle ou hexyle,
le "groupe alcoxy inférieur" est un groupe méthoxy, éthoxy, propoxy, isopropoxy, butoxy, isobutoxy, s-butoxy, t-butoxy, pentoxy ou hexyloxy,
et
le "groupe cycloalkyle inférieur" est un groupe cyclopropyle, cyclobutyle, cyclopentyle ou cyclohexyle], des sels pharmacologiquement acceptables de ceux-ci et des esters pharmacologiquement acceptables de ceux-ci.

**2.** Composition médicinale selon la revendication 1, dans laquelle un dérivé d'alcool aminé ayant la formule générale (I) est un composé ayant la formule générale (Ia) présentée ci-dessous,

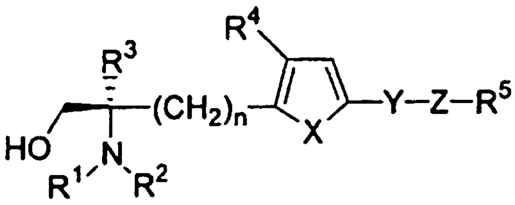

(Ia)

**3.** Composition médicinale selon la revendication 1 ou la revendication 2, dans laquelle l'inhibiteur de la HMG-CoA réductase est au moins un agent sélectionné parmi le groupe consistant en pravastatine, lovastatine, simvastatine, fluvastatine, atorvastatine, rosuvastatine et pitavastatin.

**4.** Composition médicinale selon la revendication 1 ou la revendication 2, dans laquelle l'inhibiteur de la HMG-CoA réductase est la pravastatine.

**5.** Composition médicinale selon la revendication 1 ou la revendication 2, dans laquelle l'inhibiteur de la HMG-CoA réductase est l'atorvastatine.

**6.** Composition médicinale selon l'une quelconque des revendications sélectionnées parmi les revendications 1 à 5, dans laquelle $R^1$ et $R^2$ sont un atome d'hydrogène.

**7.** Composition médicinale selon l'une quelconque des revendications sélectionnées parmi les revendications 1 à 6, dans laquelle $R^3$ est un groupe méthyle, un groupe éthyle ou un groupe hydroxyméthyle.

**8.** Composition médicinale selon l'une quelconque des revendications sélectionnées parmi les revendications 1 à 6, dans laquelle $R^3$ est un groupe hydroxyméthyle.

**9.** Composition médicinale selon l'une quelconque des revendications sélectionnées parmi les revendications 1 à 6, dans laquelle $R^3$ est un groupe méthyle.

**10.** Composition médicinale selon l'une quelconque des revendications sélectionnées parmi les revendications 1 à 9, dans laquelle $R^4$ est un atome d'hydrogène.

**11.** Composition médicinale selon l'une quelconque des revendications sélectionnées parmi les revendications 1 à 10, dans laquelle $R^5$ est un groupe phényle substitué par 1 à 3 substituants sélectionnés parmi le groupe consistant en un atome de fluor, un groupe cyano, un groupe méthyle, un groupe méthoxy, un groupe éthoxy et un groupe phényle.

**12.** Composition médicinale selon l'une quelconque des revendications sélectionnées parmi les revendications 1 à 10, dans laquelle $R^5$ est un groupe phényle substitué par 1 à 3 substituants sélectionnés parmi le groupe consistant en un groupe méthyle et un groupe méthoxy.

**13.** Composition médicinale selon l'une quelconque des revendications sélectionnées parmi les revendications 1 à 10, dans laquelle $R^5$ est un groupe phényle.

**14.** Composition médicinale selon l'une quelconque des revendications sélectionnées parmi les revendications 1 à 13, dans laquelle Y représente un groupe carbonyle.

**15.** Composition médicinale selon l'une quelconque des revendications sélectionnées parmi les revendications 1 à 14, dans laquelle Z représente une liaison simple ou un groupe alkylène $C_1$-$C_8$.

**16.** Composition médicinale selon l'une quelconque des revendications sélectionnées parmi les revendications 1 à 15, dans laquelle n représente un entier de 2.

**17.** Composition médicinale selon la revendication 1, dans laquelle un dérivé d'alcool aminé ayant la formule générale (I) est le composé suivant:

2-amino-2-méthyl-4-{1-méthyl-5-[4-(2-méthylphényl)butanoyl]pyrrol-2-yl}butan-1-ol,
2-amino-2-méthyl-4-{1-méthyl-5-[4-(3-méthylphényl)butanoyl]pyrrol-2-yl} butan-1-ol,
2-amino-2-méthyl-4-{1-méthyl-5-[4-(4-méthylphényl)butanoyl]pyrrol-2-yl}butan-1-ol,
2-amino-2-méthyl-4-{1-méthyl-5-[4-(2,3-diméthylphényl)butanoyl]pyrrol-2-yl} butan-1-ol,
2-amino-2-méthyl-4-{-méthyl-5-[4-(2,4-diméthylphényl)butanoyl]pyrrol-2-yl}butan-1-ol,
2-amino-2-méthyl-4-{1-méthyl-5-[4-(2,5-diméthylphényl)butanoyl]pyrrol-2-yl}butan-1-ol,
2-amino-2-méthyl-4-{1-méthyl-5-[4-(3,4-diméthylphényl)butanoyl]pyrrol-2-yl}butan-1-ol,
2-amino-2-méthyl-4- {1-méthyl-5- [4-(3,5 -diméthylphényl)butanoyl]pyrrol-2-yl}butan-1-ol,
2-amino-2-méthyl-4-{1-méthyl-5-[4-(3-méthyl-4-méthoxyphényl)butanoyl]pyrrol-2-yl} butan-1-ol,
2-amino-2-méthyl-4-{ 1-méthyl-5-[4-(3-méthoxy-4-méthylphényl)butanoyl]pyrrol-2-yl}butan-1-ol,
2-amino-2-méthyl-4-{1-méthyl-5-[4-(4-cyanophényl)butanoyl]pyrrol-2-yl}butan-1-ol,
2-amino-2-méthyl-4-{1-méthyl-5-[5-(2-méthylphényl)pentanoyl]pyrrol-2-yl} butan-1-ol,
2-amino-2-méthyl-4-{1-methyl-5-[5-(3-méthylphényl)pentanoyl]pyrrol-2-yl}butan-1-ol,
2-amino-2-méthyl-4-{1-méthyl-5-[5-(4-méthylphényl)pentanoyl]pyrrol-2-yl} butan-1-ol,
2-amino-2-méthyl-4-{1-méthyl-5-[5-(2,3-diméthylphényl)pentanoyl]pyrrol-2-yl}butan-1-ol,
2-amino-2-méthyl-4-{1-méthyl-5-[5-(2,4-diméthylphényl)pentanoyl]pyrrol-2-yl}butan-1-ol,
2-amino-2-méthyl-4-{1-méthyl-5-[5-(2,5-diméthylphényl)pentanoyl] pyrrol-2-yl}butan-1-ol,
2-amino-2-méthyl-4-{1-méthyl-5-[5-(3,4-diméthylphényl)pentanoyl]pyrrol-2-yl}butan-1-ol,
2-amino-2-méthyl-4-{1-méthyl-5-[5-(3,5 -diméthylphényl)pentanoyl]pyrrol-2-yl}butan-1-ol,
2-amino-2-méthyl-4-{1-méthyl-5-[5-(3-méthyl-4-méthoxyphényl)pentanoyl]pyrrol-2-yl}butan-1-ol,
2-amino-2-méthyl-4- {1-méthyl-5-[5-(3-méthoxy-4-méthylphényl)pentanoyl]pyrrol-2-yl}butan-1-ol, ou
2-amino-2-méthyl-4-{1-méthyl-5-[5-(4-cyanophényl)pentanoyl]pyrrol-2-yl}butan-1-ol,

**18.** Composition médicinale selon l'une quelconque des revendications sélectionnées parmi les revendications 1 à 17, sous forme d'un médicament combiné à utiliser dans le traitement ou la prophylaxie d'une maladie auto-immune.

**19.** Composition médicinale selon l'une quelconque des revendications sélectionnées parmi les revendications 1 à 17, sous forme d'un kit à utiliser dans le traitement ou la prophylaxie d'une maladie auto-immune, où ledit kit comprend une préparation pharmaceutique dudit au moins un inhibiteur de la HMG-CoA réductase et une préparation pharmaceutique dudit au moins un composé dérivé d'alcool aminé.

**20.** Utilisation d'une composition médicinale selon l'une quelconque des revendications sélectionnées parmi les revendications 1 à 17, dans la fabrication d'un médicament destiné au traitement ou à la prophylaxie d'une maladie auto-immune.

**21.** Utilisation d'au moins un agent sélectionné parmi le groupe d'inhibiteurs de la HMG-CoA réductase et d'au moins un composé sélectionné parmi le groupe consistant en dérivés d'alcool aminé ayant la formule générale (I), de sels pharmacologiquement acceptables de ceux-ci et d'esters pharmacologiquement acceptables de ceux-ci tels que définis dans l'une quelconque des revendications 1 à 17, dans la fabrication d'un médicament pour administrer ledit au moins un agent et ledit au moins un composé simultanément, séquentiellement ou séparément à différentes occasions, pour la prophylaxie ou le traitement d'une maladie auto-immune.

**22.** Procédé de préparation d'une composition médicinale selon l'une quelconque des revendications sélectionnées parmi les revendications 1 à 17, lequel comprend mélanger au moins un agent sélectionné parmi le groupe d'inhibiteurs de la HMG-CoA réductase et au moins un composé sélectionné parmi le groupe consistant en dérivés d'alcool aminé ayant la formule générale (I), des sels pharmaceutiquement acceptables de ceux-ci et des esters pharmaceutiquement acceptables de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002167382 A
- JP 2003267950 A
- WO 9408943 A
- EP 627406 A
- WO 9606068 A
- WO 9845249 A
- WO 03029184 A
- WO 03029205 A
- JP SHO572240 B
- US P4346227 A
- JP SHO57163374 B
- US P4231938 A
- JP SHO56122375 B
- US P4444784 A
- JP SHO60500015 B
- US P4739073 A
- JP HEI358967 B
- US P5273995 A
- JP HEI5178841 B
- US P5260440 A
- JP HEI1279866 B
- US P5854259 A
- US P5856336 A


### Non-patent literature cited in the description


- *Kidney International,* 1997, vol. 51, 94
- *Journal of Immunology,* 1996, vol. 157, 4691
- *Journal of Rheumatology,* September 2002, vol. 29 (9), 2024-6
- *Lupus.,* 2003, vol. 12 (8), 607-11